(19) Europäisches Patentamt
European Patent Office
Office européen des brevets

(11) EP 0 550 113 B1

(12) # EUROPEAN PATENT SPECIFICATION

(45) Date of publication and mention of the grant of the patent:
**15.10.1997 Bulletin 1997/42**

(51) Int Cl.[6]: **C07D 487/04**, A01N 43/90
// (C07D487/04, 249:00, 239:00)

(21) Application number: **92204097.7**

(22) Date of filing: **28.12.1992**

(54) **Triazolopyrimidine derivatives with fungicidal activity**

Triazolopyrimidinderivate mit fungizider Aktivität

Dérivés de triazolopyrimidines ayant des activités fongicides

(84) Designated Contracting States:
**AT BE CH DE DK ES FR GB GR IE IT LI LU NL PT SE**

(30) Priority: **30.12.1991 EP 91122422**

(43) Date of publication of application:
**07.07.1993 Bulletin 1993/27**

(60) Divisional application: **97105710.4**

(73) Proprietor: **SHELL INTERNATIONALE RESEARCH MAATSCHAPPIJ B.V.**
**2596 HR Den Haag (NL)**

(72) Inventors:
- **Pees, Klaus-Jürgen**
  **W-6500 Mainz (DE)**
- **Albert, Guido**
  **W-6071 Hackenheim (DE)**

(74) Representative: **Allam, Peter Clerk et al**
**LLOYD WISE, TREGEAR & CO.,**
**Commonwealth House,**
**1-19 New Oxford Street**
**London WC1A 1LW (GB)**

(56) References cited:
**EP-A- 0 071 792** **FR-A- 1 567 021**

Remarks:
The file contains technical information submitted after the application was filed and not included in this specification

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 550 113 B1

## Description

This invention relates to certain triazolopyrimidine derivatives, a process for their preparation, compositions containing such compounds and their use as fungicides.

EP-A-0071792 discloses compounds of the general formula

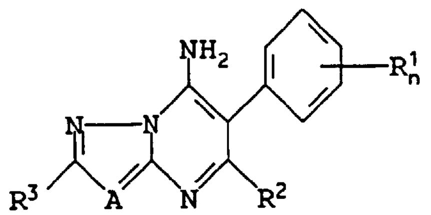

in which $R^1$ represents alkyl, halogen, alkoxy, cyano, cycloalkyl, aryl, aryloxy, arylthio, arylalkyl, arylalkyloxy or arylalkylthio each optionally substituted by halogen or alkoxy, or $R^1_n$ represents a benzene, indane or tetrahydronaphthalene ring fused with the phenyl ring, aromatic moieties in the above groups being optionally substituted by alkyl, alkoxy, halogen or cyano; n is 1 or 2; $R^2$ and $R^3$ are each hydrogen, alkyl or aryl, A represents a nitrogen atom or a $CR^4$ group; and $R^4$ is as $R^2$ but can also be halogen, cyano or alkoxycarbonyl or together with $R^3$ can form an alkylene chain containing up to 2 double bonds. The compounds are said to be active against various phytopathogenic fungi, especially those of the phycomycete class. However, evidence of fungicidal activity is only provided for 17 of the 80 disclosed compounds against Plasmopara viticola, a member of the phycomycete class of fungi.

A new class of triazolopyrimidine derivatives has now been discovered which exhibits a different spectrum of fungicidal activity, the new compounds being especially active against fungi which are members of the ascomycete class such as Venturia inaequalis, Botrytis cinerea and Alternaria solani.

According to the invention there is therefore provided a compound of the general formula

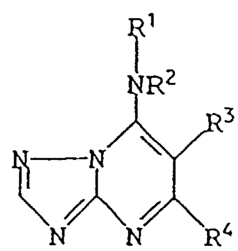

(I)

in which $R^1$ represents an optionally substituted $C_{1-12}$ alkyl, $C_{2-12}$ alkenyl, $C_{2-12}$ alkynyl, $C_{4-12}$ alkadienyl, $C_{3-8}$ cycloalkyl, $C_{4-12}$ bicycloalkyl or 3-to 6-membered heterocyclyl group; $R^2$ represents a hydrogen atom or a $C_{1-12}$ alkyl group; or $R^1$ and $R^2$ together with the interjacent nitrogen atom represent an optionally substituted 3- to 6-membered heterocyclic ring; $R^3$ represents an optionally substituted phenyl or naphthyl group; and $R^4$ represents a halogen atom or a group $-NR^5R^6$ where $R^5$ represents a hydrogen atom or an amino, $C_{1-12}$ alkyl, $C_{3-8}$ cycloalkyl or $C_{4-12}$ bicycloalkyl group and $R^6$ represents a hydrogen atom or an alkyl group; optional substituents being selected from halogen atoms, nitro, cyano, thiocyanato, cyanato, hydroxyl, $C_{1-12}$ alkyl, $C_{1-12}$ haloalkyl, $C_{1-12}$ alkoxy, $C_{1-12}$ haloalkoxy, amino, $C_{1-12}$ alkylamino, di-$C_{1-12}$ alkylamino, formyl, $C_{1-12}$ alkoxycarbonyl, carboxyl, $C_{1-12}$ alkanoyl, $C_{1-12}$ alkylthio, $C_{1-12}$ alkylsulphinyl, $C_{1-12}$ alkylsulphonyl, carbamoyl, $C_{1-12}$ alkylamido and 3- to 6-membered heterocyclyl groups and optionally substituted phenyl, phenoxy, benzyl, benzyloxy and $C_{3-8}$ cycloalkyl groups each optionally substituted by one or more halogen atoms, nitro, cyano, $C_{1-12}$ alkyl, $C_{1-12}$ haloalkyl, $C_{1-12}$ alkoxy or $C_{1-12}$ haloalkoxy groups, and additional optional substituents in the case of cycloalkyl and heterocyclyl groups being selected from saturated and unsaturated hydrocarbyl rings which may be fused with the cycloalkyl or heterocyclyl group.

When the compounds of this invention contain an alkyl, alkenyl, alkynyl or alkadienyl substituent group, this may be linear or branched and may preferably contain up to 6, and especially up to 4, carbon atoms. A cycloalkyl group may preferably contain from 3 to 6 carbon atoms. A bicycloalkyl group may preferably contain from 4 to 8 carbon atoms. A heterocyclic ring may be any 3- to 6-membered saturated or unsaturated ring system containing at least one heteroatom, 5- and 6-membered rings being especially preferred. Nitrogen-containing heterocyclic rings, such as aziridinyl, pyrrolidinyl, morphinyl and thiazolyl, are particularly preferred.

When any of the foregoing substituents are designated as being optionally substituted, typically, 0-3 substituents may be present. When any of the foregoing optional substituents represents or contain an alkyl substituent group, this may be linear or branched and may preferably contain up to 6, and especially up to 4, carbon atoms.

It is preferred that $R^1$ represents a $C_{1-12}$ alkyl, $C_{2-6}$ alkenyl, $C_{2-6}$ alkynyl, $C_{4-12}$ alkadienyl, $C_{3-8}$ cycloalkyl or $C_{4-8}$ bicycloalkyl group or a 3- to 6-membered heterocyclic ring, each group or ring being optionally substituted by one or more substituents selected from halogen atoms, nitro, cyano, hydroxyl, $C_{1-4}$ alkyl, $C_{1-4}$ haloalkyl, $C_{1-4}$ alkoxy, $C_{1-4}$ haloalkoxy, amino, $C_{1-4}$ alkylamino, di-$C_{1-4}$ alkylamino, formyl, $C_{1-4}$ alkoxycarbonyl, carboxyl, phenyl, $C_{1-4}$ haloalkylphenyl, di-$C_{1-4}$ alkoxyphenyl, furyl and dihalo-$C_{3-6}$ cycloalkyl groups or, in the case where $R^1$ represents a $C_{3-8}$ cycloalkyl group or a 3- to 6-membered heterocyclic ring, optionally ortho-fused with a benzene ring.

More preferably, $R^1$ represents a $C_{1-12}$ alkyl, $C_{2-6}$ alkenyl, $C_{2-4}$ alkynyl, $C_{4-8}$ alkadienyl, $C_{3-8}$ cycloalkyl, $C_{4-8}$ bicycloalkyl group or a 3- to 6-membered nitrogen-containing heterocyclic ring, each group or ring being optionally substituted by up to three substituents selected from halogen, especially chlorine, atoms, hydroxyl, $C_{1-4}$ alkyl, especially methyl, $C_{1-4}$ haloalkyl, especially trifluoromethyl, $C_{1-4}$ alkoxy, especially methoxy, $C_{1-4}$ haloalkoxy, especially trifluoromethoxy, phenyl, $C_{1-4}$ haloalkylphenyl, di-$C_{1-4}$alkoxyphenyl, furyl and dihalo-$C_{3-6}$ cycloalkyl groups or, in the case where $R^1$ represents a $C_{3-8}$ cycloalkyl group or a 3- to 6-membered heterocyclic ring, optionally ortho-fused with a benzene ring.

Preferably, $R^2$ represents a hydrogen atom or a $C_{1-4}$ alkyl group.

It is also preferred that $R^3$ represents a phenyl or naphthyl group, each group being optionally substituted by one or more substituents selected from halogen atoms, nitro, cyano, hydroxyl, $C_{1-12}$ alkyl, $C_{1-12}$ haloalkyl, $C_{1-12}$ alkoxy, $C_{1-12}$ haloalkoxy, amino, $C_{1-4}$ alkylamino, di-$C_{1-4}$ alkylamino, formyl, $C_{1-4}$ alkoxycarbonyl, carboxyl, phenyl, phenoxy and benzyloxy groups.

More preferably, $R^3$ represents a phenyl group optionally substituted by up to three substituents selected from halogen atoms, $C_{1-4}$ alkyl, $C_{1-4}$ haloalkyl, $C_{1-4}$ alkoxy, $C_{1-4}$ haloalkoxy, phenyl, phenoxy and benzyloxy groups, or a naphthyl group.

Preferably, $R^4$ represents a halogen atom or a group $-NR^5R^6$ where $R^5$ represents a hydrogen atom or an amino, $C_{1-4}$ alkyl, especially methyl, $C_{3-6}$ cycloalkyl or $C_{4-8}$ bicycloalkyl group and $R^6$ represents a hydrogen atom or a $C_{1-4}$ alkyl, especially methyl, group.

A particularly preferred sub-group of compounds of formula I is that in which $R^1$ represents a methyl, ethyl, propyl, heptyl, dodecyl, benzyl, dichlorocyclopropylmethyl, furylmethyl, trifluoromethylphenethyl, dimethoxyphenethyl, pentenyl, propynyl, dimethyloctadienyl, cyclopropyl, cyclopentyl, hydroxycyclopentyl, trimethylcyclopentyl, cyclohexyl, trimethylcyclohexyl, cyclooctyl, indanyl, bicycloheptyl, dichloroaziridinyl, pyrrolidinyl, morpholinyl or benzothiazolyl group; $R^2$ represents a hydrogen atom, methyl or ethyl group; or $R^1$ and $R^2$ together with the interjacent nitrogen atom represent a phenylpiperidyl group; $R^3$ represents a phenyl, fluorophenyl, chlorophenyl, bromophenyl, chloro-fluorophenyl, methylphenyl, propylphenyl, trifluoromethylphenyl, methoxyphenyl, ethoxyphenyl, dimethoxyphenyl, trimethoxyphenyl, trifluoromethoxyphenyl, biphenylyl, phenoxyphenyl, benzyloxyphenyl or naphthyl group; and $R^4$ represents a fluorine, chlorine, bromine or iodine atom or an amino, methylamino, dimethylamino, hydrazino, cyclopentylamino or bicycloheptylamino group.

The present invention also provides a process for the preparation of a compound of formula I as defined above which comprises

(a) reacting a compound of the general formula

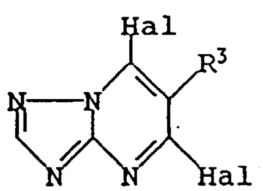

(II)

in which $R^3$ is as defined above and Hal represents a chlorine or bromine atom with a compound of the general formula

$$HNR^1R^2 \quad \text{(III)}$$

in which $R^1$ and $R^2$ are as defined above, to produce a compound of formula I in which $R^4$ represents a chlorine or bromine atom;

(b) if desired, reacting the compound of formula I formed in (a) with a fluorinating agent to produce a compound of formula I in which $R^4$ represents a fluorine atom;

(c) if desired, reacting the compound of formula I formed in (a) with a compound of the general formula

$$HNR^5R^6 \qquad (IV)$$

in which $R^5$ and $R^6$ are as defined above, to produce a compound of formula I in which $R^4$ represents a group $-NR^5R^6$; and (d) if desired, reacting a compound of formula I formed in(c) in which $R^5$ and $R^6$ both represent a hydrogen atom with diiodomethane in the presence of a diazotising agent to produce a compound of formula I in which $R^4$ represents an iodine atom.

The process of step (a) is conveniently carried out in the presence of a solvent. Suitable solvents include ethers, such as dioxane, diethyl ether and, especially, tetrahydrofuran, halogenated hydrocarbons, such as dichloromethane, and toluene. The reaction is suitably carried out at a temperature in the range from 0°C to 70°C, the preferred reaction temperature being from 10°C to 35°C. It is also preferred that the reaction is carried out in the presence of a base. Suitable bases include tertiary amines, such as triethylamine, and inorganic bases, such as potassium carbonate or sodium carbonate. Alternatively, an excess of the compound of formula III may serve as a base.

The process of step (b) is conveniently carried out in the presence of a solvent. Suitable solvents include sulpholane, dimethylformamide or a mixture of acetonitrile and a crown ether. If sulpholane or dimethylformamide is used as solvent, it is advantageous to use toluene as a co-solvent to aid dehydration of the fluorinating agent. The reaction is suitably carried out at a temperature in the range from room temperature (about 15°C) to the reflux temperature of the reaction mixture, the preferred reaction temperature being from 40°C to the reflux temperature of the reaction mixture. Suitable fluorinating agents include alkali metal fluorides, especially potassium fluoride, and antimony fluoride.

The process of step (c)is conveniently carried out in the presence of a solvent. Suitable solvents include ethers, such as dioxane, diethyl ether and tetrahydrofuran, halogenated hydrocarbons, such as dichloromethane, and, especially, toluene. The reaction is suitably carried out at a temperature in the range from 20°C to the reflux temperature of the reaction mixture, the preferred reaction temperature being from 40°C to the reflux temperature of the reaction mixture. It is also preferred that the reaction is carried out in the presence of a base. Suitable bases include tertiary amines, such as triethylamine, and inorganic bases, such as potassium carbonate or sodium carbonate. Alternatively, an excess of the compound of formula IV may serve as a base.

When $R^1$ represents the same substituent as $R^5$ and $R^2$ represents the same substituent as $R^6$ in the resultant compound of formula I, the compound of formula III will be the same as the compound of formula IV and steps (a) and (c) may therefore be performed as one step by using double the quantity of amine of formula III/IV.

The diazotising agent used in step (d) may be any alkyl ester of nitrous acid, isopentyl nitrite being especially preferred. If an alkyl ester of nitrous acid is used, this may also serve as a co-solvent with the diiodomethane. The reaction is suitably carried out at a temperature from 60°C to 120°C, the preferred reaction temperature being from 70°C to 110°C.

Compounds of formula II may be prepared by reacting a compound of the general formula

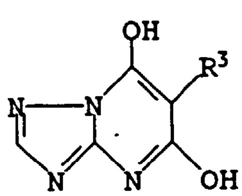

(V)

in which $R^3$ is as defined above, with a chlorinating or brominating agent, such as phosphorus oxychloride or phosphorus oxybromide.

Compounds of formula V can be prepared by reacting 3-amino-1,2,4-triazole with an appropriate malonic acid ester under alkaline conditions according to the method of Y. Makisumi, Chem. Pharm. Bull., 9, 801, (1961).

Compounds of formula III and IV are known compounds or can be prepared by processes analogous to known processes.

The compounds of general formula I have been found to have fungicidal activity. Accordingly, the invention further

provides a fungicidal composition which comprises a carrier and, as active ingredient, a compound of formula I as defined above. A method of making such a composition is also provided which comprises bringing a compound of formula I as defined above into association with at least one carrier. Such a composition may contain a single compound or a mixture of several compounds of the present invention. It is also envisaged that different isomers or mixtures of isomers may have different levels or spectra of activity and thus compositions may comprise individual isomers or mixtures of isomers.

A composition according to the invention preferably contains from 0.5 to 95% by weight of active ingredient.

A carrier in a composition according to the invention is any material with which the active ingredient is formulated to facilitate application to the locus to be treated, which may for example be a plant, seed or soil, or to facilitate storage, transport or handling. A carrier may be a solid or a liquid, including a material which is normally gaseous but which has been compressed to form a liquid, and any of the carriers normally used in formulating fungicidal compositions may be used.

Suitable solid carriers include natural and synthetic clays and silicates, for example natural silicas such as diatomaceous earths; magnesium silicates, for example talcs; magnesium aluminium silicates, for example attapulgites and vermiculites; aluminium silicates, for example kaolinites, montmorillonites and micas; calcium carbonate; calcium sulphate; ammonium sulphate; synthetic hydrated silicon oxides and synthetic calcium or aluminium silicates; elements, for example carbon and sulphur; natural and synthetic resins, for example coumarone resins, polyvinyl chloride, and styrene polymers and copolymers; solid polychlorophenols; bitumen; waxes, for example beeswax, paraffin wax, and chlorinated mineral waxes; and solid fertilisers, for example superphosphates.

Suitable liquid carriers include water; alcohols, for example isopropanol and glycols; ketones, for example acetone, methyl ethyl ketone, methyl isobutyl ketone and cyclohexanone; ethers; aromatic or araliphatic hydrocarbons, for example benzene, toluene and xylene; petroleum fractions, for example, kerosine and light mineral oils; chlorinated hydrocarbons, for example carbon tetrachloride, perchloroethylene and trichloroethane. Mixtures of different liquids are often suitable.

Fungicidal compositions are often formulated and transported in a concentrated form which is subsequently diluted by the user before application. The presence of small amounts of a carrier which is a surface-active agent facilitates this process of dilution. Thus preferably at least one carrier in a composition according to the invention is a surface-active agent. For example the composition may contain at least two carriers, at least one of which is a surface-active agent.

A surface-active agent may be an emulsifying agent, a dispersing agent or a wetting agent; it may be nonionic or ionic. Examples of suitable surface-active agents include the sodium or calcium salts of polyacrylic acids and lignin sulphonic acids; the condensation products of fatty acids or aliphatic amines or amides containing at least 12 carbon atoms in the molecule with ethylene oxide and/or propylene oxide; fatty acid esters of glycerol, sorbitol, sucrose or pentaerythritol; condensates of these with ethylene oxide and/or propylene oxide; condensation products of fatty alcohol or alkyl phenols, for example p-octylphenol or p-octylcresol, with ethylene oxide and/or propylene oxide; sulphates or sulphonates of these condensation products; alkali or alkaline earth metal salts, preferably sodium salts, of sulphuric or sulphonic acid esters containing at least 10 carbon atoms in the molecule, for example sodium lauryl sulphate, sodium secondary alkyl sulphates, sodium salts of sulphonated castor oil, and sodium alkylaryl sulphonates such as dodecylbenzene sulphonate; and polymers of ethylene oxide and copolymers of ethylene oxide and propylene oxide.

The compositions of the invention may for example be formulated as wettable powders, dusts, granules, solutions, emulsifiable concentrates, emulsions, suspension concentrates and aerosols. Wettable powders usually contain 25, 50 or 75% w of active ingredient and usually contain in addition to solid inert carrier, 3-10% w of a dispersing agent and, where necessary, 0-10% w of stabiliser(s) and/or other additives such as penetrants or stickers. Dusts are usually formulated as a dust concentrate having a similar composition to that of a wettable powder but without a dispersant, and may be diluted in the field with further solid carrier to give a composition usually containing ½-10% w of active ingredient. Granules are usually prepared to have a size between 10 and 100 BS mesh (1.676 - 0.152 mm), and may be manufactured by agglomeration or impregnation techniques. Generally, granules will contain ½-75% w active ingredient and 0-10% w of additives such as stabilisers, surfactants, slow release modifiers and binding agents. The so-called "dry flowable powders" consist of relatively small granules having a relatively high concentration of active ingredient. Emulsifiable concentrates usually contain, in addition to a solvent and, when necessary, co-solvent, 1-50% w/v active ingredient, 2-20% w/v emulsifiers and 0-20% w/v of other additives such as stabilisers, penetrants and corrosion inhibitors. Suspension concentrates are usually compounded so as to obtain a stable, non-sedimenting flowable product and usually contain 10-75% w active ingredient, 0.5-15% w of dispersing agents, 0.1-10% w of suspending agents such as protective colloids and thixotropic agents, 0-10% w of other additives such as defoamers, corrosion inhibitors, stabilisers, penetrants and stickers, and water or an organic liquid in which the active ingredient is substantially insoluble; certain organic solids or inorganic salts may be present dissolved in the formulation to assist in preventing sedimentation or as anti-freeze agents for water.

Aqueous dispersions and emulsions, for example compositions obtained by diluting a wettable powder or a concentrate according to the invention with water, also lie within the scope of the invention. The said emulsions may be of the water-in-oil or of the oil-in-water type, and may have a thick 'mayonnaise' like consistency.

The composition of the invention may also contain other ingredients, for example other compounds possessing herbicidal, insecticidal or fungicidal properties.

Of particular interest in enhancing the duration of the protective activity of the compounds of this invention is the use of a carrier which will provide a slow release of the fungicidal compounds into the environment of the plant which is to be protected. Such slow-release formulations could, for example, be inserted in the soil adjacent to the roots of a vine plant, or could include an adhesive component enabling them to be applied directly to the stem of a vine plant.

The invention still further provides the use as a fungicide of a compound of the general formula I as defined above or a composition as defined above, and a method for combating fungus at a locus, which comprises treating the locus, which may be for example plants subject to or subjected to fungal attack, seeds of such plants or the medium in which such plants are growing or are to be grown, with such a compound or composition.

The present invention is of wide applicability in the protection of crop plants against fungal attack. Typical crops which may be protected include vines, grain crops such as wheat and barley, apples and tomatoes. The duration of protection is normally dependent on the individual compound selected, and also a variety of external factors, such as climate, whose impact is normally mitigated by the use of a suitable formulation.

The invention is further illustrated by the following examples.

Example 1

Preparation of 5-chloro-6-(4-methylphenyl)-7-cyclo-pentylamino-1,2,4-triazolo[1,5-a]pyrimidine ($R^1$=cyclopentyl; $R^2$=H; $R^3$=4-methylphenyl; $R^4$=Cl)

5,7-Dichloro-6-(4-methylphenyl)-1,2,4-triazolo-[1,5-a]pyrimidine (1.8g, 6mmol) was dissolved in tetrahydrofuran. A solution of cyclopentylamine (0.51g, 6mmol) and triethylamine (0.61g, 6mmol) in tetrahydrofuran (2ml) was then added with stirring and the stirring continued for a further 3 hours at ambient temperature (20°C). The reaction mixture was then evaporated in vacuo and the residue extracted with dichloromethane and water (100ml each). The organic layer was dried over sodium sulphate and the solvent evaporated in vacuo. The residue was crystallised from ethyl acetate to give 1.7g 5-chloro-6-(4-methylphenyl)-7-cyclopentylamino-1,2,4-triazolo[1,5-a]pyrimidine as yellowish crystals, m.pt. 158°C. Yield: 87% of theoretical $^1$H-NMR:$\delta$=1.3-1.75 (2m,8H); 2.43(s,1H); 3.73(m,1H); 5.97(d,1H); 7.25(m, 4H); 8.25(s,1H) ppm

Example 2

Preparation of 5-bromo-6-phenyl-7-cyclopentylamino-1,2,4-triazolo[1,5-a]pyrimidine ($R^1$=cyclopentyl; $R^2$=H; $R^3$=phenyl; $R^4$=Br)

5,7-Dibromo-6-phenyl-1,2,4-triazolo[1,5-a]-pyrimidine (2g, 5.7mmol) was dissolved in tetrahydrofuran (40ml). A solution of triethylamine (0.61g, 6mmol) and cyclopentylamine (0.51g, 6mmol) in tetrahydrofuran (5ml) was then added whilst stirring and the stirring continued for a further 2 hours at ambient temperature (20°C). The reaction mixture was then evaporated in vacuo and the residue extracted with ethyl acetate and water (100ml each). The organic layer was dried over sodium sulphate and the solvent evaporated in vacuo. Column chromatography of the residue on a silica gel column (3.5 x 15cm) using 3:7 ethyl acetate: petroleum ether as eluant gave 0.6g 5-bromo-6-phenyl-7-cyclopentylamino-1,2,4-triazolo- [1,5-a]pyrimidine as a yellowish oil.
Yield: 28% of theoretical.
$^1$H-NMR:$\delta$=1.3-1.7(2m,8H); 3.64(m,1H); 6.05(d,1H); 7.34(m,2H); 7.50(m,3H); 8.26(s,1H) ppm

Example 3

Preparation of 5-methylamino-6-phenyl-7-cyclopentylamino-1,2,4-triazolo[1,5-a]pyrimidine ($R^1$=cyclopentyl; $R^2$=H; $R^3$=phenyl; $R^4$=$NR^5R^6$; $R^5$=$CH_3$; $R^6$=H)

A mixture of 5-chloro-6-phenyl-7-cyclopentylamino-1,2,4-triazolo[1,5-a]pyrimidine (3.1g, 10mmol), prepared by a method analogous to Example 2, methylamine (5ml), triethylamine (5ml) and toluene (50ml) was refluxed for 10 hours. After cooling, the reaction mixture was washed with water (50ml) and the organic layer separated, dried over sodium sulphate and evaporated. Recrystallisation of the solid residue from diisopropyl ether gave 2.3g 5-methylamino-6-phenyl-7-cyclopentylamino-1,2,4-triazolo[1,5-a]pyrimidine as colourless crystals, m.pt. 158-160°C.
Yield: 75% of theoretical

$^1$H-NHR: $\delta$ =1.25-1.7(mm,8H); 2.95(d,3H); 6.16(m,1H); 7.34(m,1H); 8.32(d,1H); 7.3-7.5(m,5H); 8.03(s,1H)

Example 4

Preparation of 5-fluoro-6-(4-methoxyphenyl-7-cyclopentylamino-1,2,4-triazolo[1,5-a]pyrimidine ($R^1$=cyclopentyl; $R^2$=H; $R^3$=4-methoxyphenyl; $R^4$=F)

Potassium fluoride (3.1g, 0.05mol) was suspended in a mixture of dry sulpholane (60ml) and toluene (20ml) and the mixture was then refluxed for 6 hours over a water separator. 5-Chloro-6-(4-methoxyphenyl)-7-cyclopentylamino-1,2,4-triazolo[1,5-a]pyrimidine (8.5g, 0.025mol), obtained by a method analogous to that of Example 1 above, was added at room temperature and an azeotrope of sulpholane and toluene distilled off until the reaction temperature reached 200°C. The reaction mixture was then kept at this temperature for 3 days before cooling to room temperature and then pouring into water (600ml). The mixture was then filtered and the precipitate washed with water. The precipitate was then dissolved in dichloromethane, extracted twice with water, dried with sodium sulphate and the solvent was distilled off in vacuo. The residue was then washed twice with warm diethyl ether, the ether fraction was decanted off and then dried in vacuo. Flash column chromatography on silica gel using a mixture of petroleum ether and ethyl ethanoate as eluant yielded 4.5g 5-fluoro-6-(4-methoxyphenyl)-7-cyclopentylamino-1,2,4-triazolo [1,5-a]pyrimidine as a colourless crystalline solid, m.pt. 124°C.
Yield: 55% of theoretical.

Example 5

Preparation of 5-iodo-6-(2-chlorophenyl)-7-cyclopentylamino -1,2,4-triazolo[1,5-a]pyrimidine ($R^1$=cyclopentyl; $R^2$=H; $R^3$=4-methoxyphenyl; $R^4$=I

5-Amino-6-(2-chlorophenyl)-7-cyclopentylamino 1,2,4-triazolo[1,5-a]pyrimidine (3.3g, 10mmol), obtained by a method analogous to that of Example 3 above, and diiodomethane (50ml) were mixed together. Isopentyl nitrite (20ml) was added under nitrogen and the reaction mixture heated for 3 hours at 90°C. The reaction mixture was then cooled to room temperature and filtered. The solvent was distilled off in vacuo and the residue was purified by flash column chromatography on silica gel using 7:3 petroleum ether:ethyl ethanoate as eluant to yield 1.33g 5-iodo-6-(2-chlorophenyl)-7-cyclopentylamino-1,2,4-triazolo-[1,5-a]pyrimidine as colourless crystals, m.pt. 150°C. Yield: 30.3% of theoretical.

Examples 6 - 114

By processes similar to those described in Examples 1 to 5 above, further compounds according to the invention were prepared as detailed in Table I below. In this table the compounds are identified by reference to formula I. Melting point, NMR and C,H,N analysis data for the compounds of Examples 6 to 114 are given in Table IA below.

TABLE I

| Ex. No. | $R^1$ | $R^2$ | $R^3$ | $R^4$ |
|---|---|---|---|---|
| 6 | cyclopentyl | H | $2-OCH_3$ phenyl | Cl |
| 7 | " | " | $3-OCH_3$ phenyl | " |
| 8 | " | " | $4-OC_2H_5$ phenyl | " |
| 9 | 2-OH cyclopentyl | " | $4-OCH_3$ phenyl | " |
| 10 | $2,4,4-(CH_3)_3$ cyclopentyl | " | phenyl | " |
| 11 | cyclooctyl | " | $4-OCH_3$ phenyl | " |
| 12 | 4-phenylpiperidyl | | phenyl | " |
| 13 | $2,4-(CH_3)_2$ pent-3-yl | | " | " |
| 14 | cyclopentyl | " | $4-OCH_3$ phenyl | cyclopentyl amino |
| 15 | " | " | " | $-NHCH_3$ |
| 16 | $-CH(CH_3)_2$ | " | $4-OC_2H_5$ phenyl | Cl |
| 17 | $2,2,5-(CH_3)_3$ cyclohexyl cyclohexyl | " | phenyl | " |
| 18 | indan-2-yl | " | phenyl | " |
| 19 | $-CH_3$ | $-CH_3$ | 3-Cl phenyl | " |
| 20 | $-CH(CH_3)_2$ | H | $4-CH_3$ phenyl | " |
| 21 | cyclopentyl | " | $4-OCH_3$ phenyl | " |
| 22 | cyclohexyl | " | phenyl | " |
| 23 | $C_{12}H_{25}$ | " | " | " |
| 24 | cyclopentyl | " | phenyl | " |
| 25 | cyclopropyl | " | " | " |
| 26 | cyclopentyl | " | $3-CF_3$ phenyl | " |
| 27 | " | " | $4-{}^{i}C_3H_7$ phenyl | " |
| 28 | " | " | $4-OCF_3$ phenyl | " |
| 29 | " | " | naphth-2-yl | " |
| 30 | " | " | $3,4-(OCH_3)_2$ phenyl | " |

TABLE I (continued)

| Ex. No. | $R^1$ | $R^2$ | $R^3$ | $R^4$ |
|---|---|---|---|---|
| 31 | cyclopentyl | H | 2-Cl phenyl | Cl |
| 32 | " | " | 4-F phenyl | " |
| 33 | " | " | 4-biphenylyl | " |
| 34 | $-CH_2C\equiv CH$ | " | phenyl | " |
| 35 | benzyl | " | " | " |
| 36 | cyclopentyl | " | 2-Br phenyl | " |
| 37 | $-CH(CH_3)_2$ | " | " | " |
| 38 | bicyclo[2.2.1]hept-2-yl | " | " | " |
| 39 | cyclopentyl | " | 2-F phenyl | " |
| 40 | $-CH(CH_3)_2$ | " | " | " |
| 41 | " | " | naphth-2-yl | " |
| 42 | " | " | 2-Cl phenyl | " |
| 43 | " | " | 4-F phenyl | " |
| 44 | $-CH_2CH{=}C(CH_3)_2$ | " | phenyl | " |
| 45 | $-CH_3$ | $-CH_3$ | $4-OCH_3$ phenyl | " |
| 46 | $-CH_2CH{=}C(CH_3)CH_2CH_2CH{=}C(CH_3)_2$ | -H | $4-CH_3$ phenyl | " |
| 47 | " | " | $4-OCH_3$ phenyl | " |
| 48 | $-CH_3$ | $-CH_3$ | " | $-N(CH_3)_2$ |
| 49 | fur-2-ylmethyl | -H | phenyl | -Cl |
| 50 | benzothiazol-2-yl | " | " | " |
| 51 | morpholin-4-yl | " | " | " |
| 52 | 2-OH cyclopentyl | " | " | " |
| 53 | cyclopentyl | " | $4-OC_6H_5$ phenyl | " |
| 54 | $-CH(CH_3)_2$ | " | $3-CF_3$ phenyl | " |
| 55 | " | " | $4-{}^{i}C_3H_7$ phenyl | " |
| 56 | " | " | $4-CF_3O$ phenyl | " |
| 57 | " | " | $4-OC_6H_5$ phenyl | " |
| 58 | " | " | 4-biphenylyl | " |
| 59 | " | " | $3,4-(OCH_3)_2$ phenyl | " |
| 60 | cyclopentyl | " | $4-OCH_2C_6H_5$ phenyl | " |

TABLE I (continued)

| Ex. No. | $R^1$ | $R^2$ | $R^3$ | $R^4$ |
|---|---|---|---|---|
| 61 | $-CH(CH_3)_2$ | -H | $4-OCH_2C_6H_5$ phenyl | -Cl |
| 62 | bicyclo[2.2.1]hept-2-yl | " | $4-OCH_3$ phenyl | " |
| 63 | " | " | 2-Cl phenyl | " |
| 64 | cyclopentyl | " | 4-Br phenyl | " |
| 65 | $-CH(CH_3)_2$ | " | " | " |
| 66 | bicyclo[2.2.1]hept-2-yl | " | " | " |
| 67 | " | " | 3-Br phenyl | " |
| 68 | cyclopentyl | " | " | " |
| 69 | bicyclo[2.2.1]hept-2-yl | " | 2-F phenyl | " |
| 70 | cyclopentyl | " | 3-F phenyl | " |
| 71 | $-CH(CH_3)_2$ | " | " | " |
| 72 | bicyclo[2.2.1]hept-2-yl | " | " | " |
| 73 | cyclopentyl | " | $2-OCH_2C_6H_5$ phenyl | " |
| 74 | $-CH(CH_3)_2$ | " | " | " |
| 75 | bicyclo[2.2.1]hept-2-yl | " | " | " |
| 76 | cyclopentyl | " | $2,3-(OCH_3)_2$ phenyl | " |
| 77 | $-CH(CH_3)_2$ | " | " | " |
| 78 | bicyclo[2.2.1]hept-2-yl | " | " | " |
| 79 | $-CH_2CH_2-(3-CF_3$ phenyl) | " | $4-OCH_3$ phenyl | " |
| 80 | " | " | 2-Cl phenyl | " |
| 81 | 2,2-dichloroaziridin-1-yl | " | $4-OCH_3$ phenyl | " |
| 82 | " | " | 2-Cl phenyl | " |
| 83 | pyrrolidin-1-yl | " | " | " |
| 84 | bicyclo[2.2.1]hept-2-yl | " | $4-OCH_3$ phenyl | -NH-bicyclo-[2.2.1]-hept-2-yl |
| 85 | " | " | 2-Cl phenyl | " |

TABLE I (continued)

| Ex. No. | $R^1$ | $R^2$ | $R^3$ | $R^4$ |
|---|---|---|---|---|
| 86 | cyclopentyl | " | " | $-NH-NH_2$ |
| 87 | cyclopentyl | " | $4-OCH_3$ phenyl | - |
| 88 | bicyclo[2.2.1]hept-2-yl | " | 2-Cl phenyl | " |
| 89 | 2,2-dichlorocycloprop-1-yl | " | $3,4,5-(OCH_3)_3$ phenyl | -Cl |
| 90 | cyclopentyl | " | 2-F phenyl | $-NH-NH_2$ |
| 91 | bicyclo[2.2.1]hept-2-yl | " | phenyl | -Br |
| 92 | $-CH(CH_3)_2$ | " | " | " |
| 93 | 2,2-dichlorocycloprop-1-yl | " | " | " |
| 94 | $-CH_3$ | " | 2-Cl phenyl | -Cl |
| 95 | " | $-CH_3$ | " | " |
| 96 | $-C_2H_5$ | -H | " | " |
| 97 | $-CH_3$ | " | 2-F phenyl | " |
| 98 | " | $-CH_3$ | " | " |
| 99 | $-C_2H_5$ | -H | " | " |
| 100 | cyclopentyl | " | $4-OCH_3$ phenyl | $-NH_2$ |
| 101 | " | " | 2-Cl phenyl | " |
| 102 | " | " | 2-Cl,6-F phenyl | -Cl |
| 103 | $-CH(CH_3)_2$ | " | " | " |
| 104 | bicyclo[2.2.1]hept-2-yl | " | 2-Cl,6-F phenyl | " |
| 105 | $-CH_2CH_2-(3,4-(OCH_3)_2phenyl)$ | " | " | " |
| 106 | $-CH_3$ | $-CH_3$ | " | " |
| 107 | bicyclo[2.2.1]hept-2-yl | -H | 2-Cl phenyl | $-NH_2$ |
| 108 | $-C_2H_5$ | $-C_2H_5$ | 2-Cl,6-F phenyl | -Cl |
| 109 | " | " | 2-F phenyl | " |
| 110 | " | " | 2-Br phenyl | " |
| 111 | " | " | 2-Cl phenyl | " |
| 112 | " | " | $4-OCH_3$ phenyl | " |
| 113 | cyclopentyl | -H | " | I |
| 114 | bicyclo[2.2.1]hept-2-yl | " | 2-Cl phenyl | " |

TABLE IA

| Ex. No. | $^1$H-NMR(ppm) | M.pt. (°C) | Elemental Analysis C Calc. | C Found | H Calc. | H Found | N Calc. | N Found |
|---|---|---|---|---|---|---|---|---|
| 6 | 1.3-1.7(m,8H), 3.7 (m,1H), 3.75(s,3H), 6.1(d,1H), 7.0 (m,2H), 7.25(m,1H), 7.48(dt,1H), 8.25 (s,1H) | 121 | | | | | | |
| 7 | 1.35-1.78(m,8H), 3.75(m,1H), 3.85 (s,3H), 6.05(d,1H), 6.95(m,3H), 7.88 (dt,1H), 8.27(s,1H) | 110 | | | | | | |
| 8 | 1.3-1.8(2m,8H), 1.45(t,3H), 3.78 (m,1H), 4.08(q,2H), 6.05(d,1H), 7.00 (m,2H), 7.25(m,2H), 8.25(s,1H) | 118 | | | | | | |
| 9 | 1.3-2.03(3m,6H), 3.25(m,1H), 3.86 (s,3H), 5.75(d,1H), 7.02(m,2H), 7.27 (dd,1H), 7.44(dd,1H), 8.20(s,1H) | 148 | | | | | | |
| 10 | 0.5-1.8(mm,13H), 6.06(d,1H), 7.83 (m,2H), 7.45(m,3H), 8.28(s,1H) | 124-130 | | | | | | |

TABLE IA (continued)

| Ex. No. | $^1$H-NMR(ppm) | M.pt. (°C) | Elemental Analysis C Calc. | C Found | H Calc. | H Found | N Calc. | N Found |
|---|---|---|---|---|---|---|---|---|
| 11 | 1.1-1.75(m,14H), 3.55(m,1H), 3.9 (s,3H), 6.05(d,1H), 7.05(dd,2H), 7.25 (dd,2H) | 118 | | | | | | |
| 12 | 1.7-1.9(m,4H), 2.6 (m,1H), 2.75(m,2H), 4.84(m,2H), 7.1-7.5 (mm,10H), 8.4(s,1H) | 168 | | | | | | |
| 13 | 0.5-2.7(mm,15H), 6.45(m,1H), 7.2-7.6 (mm,5H), 8.32(s,1H) | oil | | | | | | |
| 14 | 1.07-2.05(mm,16H), 3.37(m,1H), 3.86 (s,3H), 4.30(d,1H), 4.45(m,1H), 4.97 (d,1H), 7.0(dd,2H), 7.28(dd,2H), 8.0 (s,1H) | oil | | | | | | |
| 15 | 1.3-1.9(mm,8H), 2.95(d,3H), 3.87 (s,3H), 4.40(d,1H), 5.50(d,1H), 7.00 m,2H), 7.24(m,2H), 8.03(s,1H) | 180 | | | | | | |
| 16 | 1.03(2s,6H), 1.46 (t,3H), 3.67(m,1H), 4.06(q,2H), 5.85 (d,1H), 7.0(d,2H), 7.23(d,2H), 8.26 (s,1H) | 122 | | | | | | |

TABLE IA (continued)

| Ex. No. | $^1$H-NMR(ppm) | M.pt. (°C) | Elemental Analysis C Calc. | C Found | H Calc. | H Found | N Calc. | N Found |
|---|---|---|---|---|---|---|---|---|
| 17 | 0.5-1.7(mm,17H), 3.25(m,1H), 5.95 (d,1H), 7.34(m,2H), 7.47(m,3H), 8.28 (s,1H) | 130 | | | | | | |
| 18 | 1.2-1.9(m,1H), 2.1-2.25(m,1H), 2.55-2.7(m,1H), 2.86-2.96 (m,1H), 5.05(s,1H), 6.29(d,1H), 7.12-7.57 (2m,4H) | oil | | | | | | |
| 19 | | 177-179 | 50.66 | 50.60 | 3.59 | 3.83 | 22.72 | 22.66 |
| 20 | | 113 | 59.69 | 59.59 | 5.34 | 5.33 | 23.20 | 23.33 |
| 21 | 1.3-1.8(2m,8H), 3.8(m,1H), 3.90 (s,3H), 6.03(d,1H), 6.98(dd,2H), 7.28 (dd,2H), 8.27(s,1H) | 140 | 59.38 | 59.42 | 5.27 | 5.39 | 20.37 | 20.39 |
| 22 | | 130-132 | 62.28 | 62.26 | 5.58 | 5.48 | 21.36 | 21.31 |
| 23 | | 92-94 | 66.76 | 66.74 | 7.79 | 7.78 | 16.91 | 16.79 |
| 24 | 1.3-1.8(2m,8H), 3.63(m,1H), 6.08 (d,1H), 7.35(m,2H), 7.56(m,3H), 8.30 (s,1H) | 125 | 61.23 | 61.15 | 5.13 | 5.16 | 22.32 | 22.33 |
| 25 | | 175 | 58.84 | 58.69 | 4.23 | 4.22 | 24.51 | 24.47 |

TABLE IA (continued)

| Ex. No. | $^1$H-NMR(ppm) | M.pt. (°C) | Elemental Analysis C Calc. | C Found | H Calc. | H Found | N Calc. | N Found |
|---|---|---|---|---|---|---|---|---|
| 26 | 1.05-1.86(m,8H), 2.6(s,1H), 3.4-3.7 (2m,2H), 7.60-8.0 (m,4H), 8.6(s,1H) | 165 | | | | | | |
| 27 | 1.05-1.7(mm,14H), 2.6(s,2H), 3.05 (m,1H), 3.46(m,1H), 7.38-7.44(2m,4H), 8.68(s,1H) | 112 | | | | | | |
| 28 | 1.05-1.7(mm,8H), 2.6(s,1H), 3.6 (m,1H), 7.60(dd,2H), 7.68(dd,2H), 8.62 s,1H) | oil | | | | | | |
| 29 | 1.0-1.7(mm,8H), 2.6(m,1H), 3.46 (s,1H), 7.68(m,3H), 8.1(m,4H), 8.70 (s,1H) | 107 | | | | | | |
| 30 | 1.1-1.8(mm,8H), 3.48(s,1H), 3.84 (d,3H), 3.94(d,3H), 6.9-7.35(m,3H), 8.63(s,1H) | oil | | | | | | |
| 31 | 1.34-1.8(m,8H), 3.55(m,1H), 6.22 (d,1H), 7.48-7.55 (m,4H), 8.32(s,1H) | 145 | | | | | | |

TABLE IA (continued)

| Ex. No. | $^1$H-NMR(ppm) | M.pt. (°C) | Elemental Analysis C Calc. | C Found | H Calc. | H Found | N Calc. | N Found |
|---|---|---|---|---|---|---|---|---|
| 32 | 1.23-1.75(m,8H), 3.46(s,1H), 3.72 (m,1H), 7.45(m,2H), 7.65(m,2H), 8.65 (s,1H) | oil | | | | | | |
| 33 | 1.05-1.8(m,8H), 3.48(s,1H), 3.74 (m,1H), 7.48-8.0 (m,9H), 8.68(s,1H) | 65 | | | | | | |
| 34 | | 126 | 59.26 | 59.48 | 3.55 | 3.78 | 24.68 | 24.68 |
| 35 | | 105 | 64.37 | 65.69 | 4.20 | 4.36 | 20.85 | 19.50 |
| 36 | | 160 | | | | | | |
| 37 | | 60 | | | | | | |
| 38 | | 140 | | | | | | |
| 39 | | 97 | | | | | | |
| 40 | | 142 | | | | | | |
| 41 | | 150 | | | | | | |
| 42 | | 128 | | | | | | |
| 43 | | 99 | | | | | | |
| 44 | | 95 | | | | | | |

TABLE IA (continued)

| Ex. No. | $^1$H-NMR(ppm) | M.pt. (°C) | Elemental Analysis C Calc. | C Found | H Calc. | H Found | N Calc. | N Found |
|---|---|---|---|---|---|---|---|---|
| 45 | | 134 | 56.68 | 56.62 | 5.07 | 5.08 | 22.04 | 22.03 |
| 46 | ($CDCl_3$): 1.4(s,3H); 1.55(s,3H); 1.7(s,3H); 1.9(m,2H); 2.4(s,3H); 3.5(m,2H); 5.0(m,1H); 5.1(m,1H); 6.0(m,1H); 7.25(m,4H); 8.3(s,1H) | | | | | | | |
| 47 | ($CDCl_3$): 1.4(s,3H); 1.5(s,3H); 1.6(s,3H); 1.9(m,2H); 3.5(m,2H); 3.8(s,3H); 5.0(m,1H); 5.1(m,1H); 5.9(t,1H); 6.9(d,2H); 7.2(d,2H); 8.2(s,1H) | | | | | | | |
| 48 | | 194 | | | | | | |
| 49 | | 100 | | | | | | |
| 50 | | 198 | | | | | | |
| 51 | ($CDCl_3$): 2.3(m,2H); 2.6(m,4H); 3.5(m,2H); 7.2(s,1H); 7.25(m,2H); 7.4(m,3H); 8.4(s,1H) | | | | | | | |
| 52 | | 162 (decomp) | | | | | | |

TABLE IA (continued)

| Ex. No. | $^{1}$H-NMR(ppm) | M.pt. (°C) | Elemental Analysis C Calc. | C Found | H Calc. | H Found | N Calc. | N Found |
|---|---|---|---|---|---|---|---|---|
| 53 | (dmso-$d_6$): 1.2-1.4(m,2H); 1.6-1.8(m,6H); 3.7(m,1H); 7.1-7.3 (m,5H); 7.5-7.6 (m,4H); 7.7(d,1H); 8.6(s,1H) | | | | | | | |
| 54 | | 138 | | | | | | |
| 55 | | 100 | | | | | | |
| 56 | | 108 | | | | | | |
| 57 | | 145 | | | | | | |
| 58 | | 65-70 | | | | | | |
| 59 | | 150 | | | | | | |
| 60 | | 138 | | | | | | |
| 61 | (dmso-$d_6$): 1.1(d,6H); 3.6(m,1H); 5.3(s,2H); 7.2(d,1H); 7.4-7.6(m,7H); 8.6(s,1H) | | | | | | | |
| 62 | (acetone-$d_6$): 0.5(m,1H); 0.9 (m,1H); 1.1(d,1H); 1.2-1.6(m,6H); 1.80(m,1H); 2.2(m,2H); 3.3(m,1H);3.9(s,3H); 6.3(d,1H); 7.1(m,2H); 7.4-7.5(m,2H); 8.4(s,1H) | | | | | | | |

TABLE IA (continued)

| Ex. No. | 'H-NMR(ppm) | M.pt. (°C) | Elemental Analysis C Calc. | C Found | H Calc. | H Found | N Calc. | N Found |
|---|---|---|---|---|---|---|---|---|
| 63 | (acetone-$d_6$): ppm: 0.2-0.4(m,1H); 0.9(m,1H); 1.1(m,1H); 1.2-1.6(m,5H); 2.20 (m,2H); 3.2(m,1H); 6.6(t,1H); 7.5-7.8 (m,4H); 8.4(s,1H) | | | | | | | |
| 64 | | 167 | | | | | | |
| 65 | | 80 | | | | | | |
| 66 | | 180 | | | | | | |
| 67 | | 140 | | | | | | |
| 68 | | 150 | | | | | | |
| 69 | | 174 | | | | | | |
| 70 | | 130 | | | | | | |
| 71 | | 130 | | | | | | |
| 72 | | 170 | | | | | | |
| 73 | (dmso-$d_6$): 1.3-1.5(m,4H); 1.5-1.7(m,4H); 3.7 (m,1H); 5.1(s,2H); 6.1(d,1H); 7.05 (m,2H); 7.3(m,6H); 7.4(t,1H); 8.3(s,1H) | | | | | | | |
| 74 | ($CDCl_3$): 1.1(m,6H); 3.6(m,1H); 5.1(s,1H); 5.95(d,1H); 7.3-7.3(m,6H); 7.4 (t,1H); 8.3(s,1H) | | | | | | | |

TABLE IA (continued)

| Ex. No. | $^1$H-NMR(ppm) | M.pt. (°C) | Elemental Analysis C Calc. | C Found | H Calc. | H Found | N Calc. | N Found |
|---|---|---|---|---|---|---|---|---|
| 75 | ($CDCl_3$): 0.1(m,1H); 0.7 (m,1H); 0.8-1.3(m,7H); 2.0 (m,1H); 3.0(m,1H); 4.9(s,1H); 5.9(m,1H); 6.8-6.9(m,2H); 7.0-7.15(m,6H); 7.25(m,1H); 8.05(s,1H) | | | | | | | |
| 76 | | 162 | | | | | | |
| 77 | | 141 | | | | | | |
| 78 | | 73 (amorph.) | | | | | | |
| 79 | | 140 | | | | | | |
| 80 | | 112 | | | | | | |
| 81 | ($CDCl_3$): 1.2(t,1H); 1.6 (t,1H); 1.8(m,1H); 3.1(m,1H); 3.8(m,1H); 3.9(s,3H); 6.25(t,1H); 7.0(d,2H); 7.3(d,2H); 8.3(s,1H) | | | | | | | |
| 82 | | 68-78 (amorph) | | | | | | |
| 83 | | 240 | | | | | | |
| 84 | | 258 | | | | | | |
| 85 | | 170 | | | | | | |

TABLE IA (continued)

| Ex. No. | $^1$H-NMR(ppm) | M.pt. (°C) | Elemental Analysis C Calc. | C Found | H Calc. | H Found | N Calc. | N Found |
|---|---|---|---|---|---|---|---|---|
| 86 | (dmso-$d_6$): 1.2-1.4(m,2H); 1.4-1.7(m,6H); 3.4(m,1H); 4.4(m,2H); 5.8(m,1H); 6.5(d,1H); 6.9(m,1H); 7.6(m,3H); 7.8(d,1H); 8.3(s,1H) | | | | | | | |
| 87 | | 121 | | | | | | |
| 88 | (dmso-$d_6$): 0-0.2(m,1H); 0.8(m,1H); 1.0-1.6 (m,6H); 2.0(m,1H); 2.2(m,1H); 2.7(m,1H); 6.1(d,1H); 7.5(m,3H); 7.6((d,1H); 8.2(s,1H) | | | | | | | |

TABLE IA (continued)

| Ex. No. | $^1$H-NMR(ppm) | M.pt. (°C) | Elemental Analysis C Calc. | C Found | H Calc. | H Found | N Calc. | N Found |
|---|---|---|---|---|---|---|---|---|
| 89 | | 205 | | | | | | |
| 90 | (dmso-$d_6$): 1.1-1.3(m,2H); 1.4-1.7(m,6H); 3.4(m,1H); 4.0-4.6 (broad,2H); 6.4 (d,1H); 7.0(broad, 1H); 7.2(m,1H); 7.3-7.5(m,2H); 7.6(m,1H); 8.2(s,1H) | | | | | | | |
| 91 | (dmso-$d_6$): 0.1(m,1H); 0.7 (m,1H); 1(m,1H); 1.1-1.6(m,5H); 2.0(m,1H); 2.1 (m,1H); 3.0(m,1H); 6.9(d,1H); 7.4-7.6(m,5H); 8.6 (s,1H) | | | | | | | |
| 92 | | 148 | | | | | | |
| 93 | | 116 | | | | | | |
| 94 | | 112 | | | | | | |
| 95 | | 150 | | | | | | |
| 96 | | 154 | | | | | | |
| 97 | | 210 | | | | | | |
| 98 | | 163 | | | | | | |
| 99 | | 160 | | | | | | |
| 100 | | 213 | | | | | | |
| 101 | | 230 | | | | | | |

TABLE IA (continued)

| Ex. No. | $^1$H-NMR(ppm) | M.pt. (°C) | Elemental Analysis C Calc. | C Found | H Calc. | H Found | N Calc. | N Found |
|---|---|---|---|---|---|---|---|---|
| 102 | | 102 | | | | | | |
| 103 | | 140 | | | | | | |
| 104 | | 185 | | | | | | |
| 105 | | 143 | | | | | | |
| 106 | | 138 | | | | | | |
| 107 | | 275 | | | | | | |
| 108 | | 163 | | | | | | |
| 109 | | 150 | | | | | | |
| 110 | (dmso-$d_6$): 1.0(t,6H); 3.2(m,2H); 3.5(m,2H); 7.6(m,2H); 7.9(d,1H); 8.6(s,1H) | | | | | | | |
| 111 | (dmso-$d_6$): 1.0(t,6H); 3.2(q,4H); 7.3(m,2H); 7.5(m,2H); 8.6(s,1H) | | | | | | | |
| 112 | (dmso-$d_6$): 1.0(t,6H); 3.2(q,4H); 3.8(s,1H); 7.1(d,2H); 7.4(d,2H); 8.6(s,1H) | | | | | | | |
| 113 | | 200 | | | | | | |
| 114 | | 84 (amorph.) | | | | | | |

Example 115

Fungicidal activity against Venturia inaequalis on Malus sp.

Apple cuttings of the variety Morgenduft, which are about 6 weeks old, were treated with a solution of the test compound (400 ppm) in water/acetone/Triton X or water/methanol/Triton X. After 24 hours, the plants were infected with a conidia suspension of Venturia inaequalis (about 50,000 conidia/ml), incubated in a dark climatic chamber at a relative humidity of 100% for 48 hours and then kept at a relative humidity of 95-99% and temperature of 18-20°C during the day and 13°C during the night for about 14 days. The extent of infection was assessed according to the following scheme:-

0 = no infection
1 = 1-10% infection
2 = 11-40% infection

3 = 41-100% infection

The results of these tests are set out in Table II below:-

TABLE II

| Example No. | Activity |
|---|---|
| 1 | 1 |
| 2 | 0 |
| 3 | 2.3 |
| 6 | 1.8 |
| 7 | 1 |
| 8 | 1 |
| 10 | 2.8 |
| 11 | 1.3 |
| 12 | 2.3 |
| 13 | 2 |
| 14 | 2.7 |
| 15 | 2.7 |
| 16 | 0 |
| 18 | 2.4 |
| 19 | 3. |
| 20 | 1 .4 |
| 21 | 0 |

TABLE II (continued)

| Example No. | Activity |
|---|---|
| 22 | 2.5 |
| 23 | 3 |
| 24 | 1 |
| 26 | 2.5 |
| 27 | 2.3 |
| 28 | 1.8 |
| 29 | 1.3 |
| 30 | 2.3 |
| 31 | 0 |
| 32 | 0 |
| 33 | 1.5 |
| 36 | 1.3 |
| 37 | 0 |
| 38 | 1.0 |
| 39 | 0 |
| 40 | 0 |
| 42 | 0 |
| 45 | 1.0 |
| 46 | 2.8 |
| 47 | 2.9 |
| 48 | 2.9 |
| 49 | 2.9 |
| 50 | 2.5 |
| 51 | 2.8 |
| 54 | 2.5* |
| 55 | 1.5* |
| 56 | 1.5* |
| 57 | 2.3* |

* signifies concentration of test compound = 200 ppm.

TABLE II (continued)

| Example No. | Activity |
|---|---|
| 58 | 1.8* |
| 59 | 1.5* |
| 60 | 0 |
| 61 | 0 |
| 62 | 0 |
| 63 | 0 |
| 69 | 0 |
| 70 | 1.3 |
| 72 | 2.8 |
| 79 | 0.8 |
| 80 | 2.3 |
| 81 | 2.3 |
| 82 | 1.6 |
| 85 | 1.3 |
| 88 | 2.0 |
| 102 | 0 |
| 103 | 0 |
| 104 | 0 |

* signifies concentration of test compound - 200 ppm.

Example 116

Determination of MIC-Values of compounds against various phytopathogenic fungi

The MIC (Minimum Inhibition Concentration)-values were determined by serial dilution tests using 48-well microtitre plates. The dilution of the test compounds in the nutrient solution and the distribution to the wells were carried out by a TECAN RSP 5000 robotic sample processor.

The compounds were diluted to the following concentrations: 100,50, 25, 12.5, 6.25, 3.13, 1.56, 0.78, 0.39, 0.20, 0.10 and 0.05 µg/ml.

For preparation of the nutrient solution, V8 juice (Trade Mark) was neutralised with calcium carbonate and centrifuged. The supernatant was diluted with distilled water (1:5) to the final concentration.

The fungi (Alternaria solani, Botrytis cinerea, Septoria nodorum) were added into the wells as a droplet of spore suspension. The microtitre plates were then incubated at 20°C for 6-8 days. The MIC-value was determined by visual inspection of the plates. In the case of Alternaria solani and Botrytis cinerea, the lowest concentration in the dilution series without mycelial growth was defined to be the MIC-value. For Septoria nodorum, no MIC-value but only a strong inhibition of growth was regularly observed.

The results of these test are set out in Table III below:-

TABLE III

| Example No. | MIC-value (ppm) | | |
|---|---|---|---|
| | Botrytis cinerea | Alternaria solani | Septoria nodorum |
| 1 | 12.5 | 1.56 | |
| 5 | 6.25 | 3.13 | |
| 6 | 25.0 | | |
| 7 | 6.25 | | |
| 8 | 3.13 | | |
| 9 | | 12.5 | |
| 17 | >100.0 | | |
| 18 | 50.0 | >100.0 | |
| 19 | 100.0 | 100.0 | |

TABLE III (continued)

| Example No. | MIC-value (ppm) Botrytis cinerea | Alternaria solani | Septoria nodorum |
|---|---|---|---|
| 20 | 12.5 | 25.0 | |
| 21 | 1.56 | 0.39 | > 12.5 |
| 23 | >100.0 | | |
| 24 | 6.25 | 0.78 | > 3.13 |
| 25 | 50.0 | | |
| 27 | | | > 12.5 |
| 28 | | | > 3.13 |
| 31 | 0.78 | 0.39 | > 0.39 |
| 32 | 6.25 | 1.56 | > 3.13 |
| 34 | 50.0 | | |
| 35 | >100.0 | | |
| 36 | | 0.78 | |
| 37 | 12.50 | 25.00 | |
| 38 | 25.00 | 0.39 | |
| 39 | 3.13 | 0.78 | |
| 40 | 25.00 | 12.50 | |
| 42 | 6.25 | 12.50 | |
| 45 | 12.50 | 12.50 | |
| 62 | 6.25 | 0.05 | |
| 63 | 3.13 | 0.05 | |
| 64 | | 3.13 | |
| 69 | 3.13 | 0.20 | |
| 70 | | 3.13 | |
| 72 | | 1.56 | |
| 81 | 12.50 | 3.13 | |
| 82 | 6.25 | 1.56 | |
| 84 | | 6.25 | |

TABLE III (continued)

| Example No. | MIC-value (ppm) Botrytis cinerea | Alternaria solani | Septoria nodorum |
|---|---|---|---|
| 85 | 25.00 | 1.56 | |
| 88 | | 12.50 | |
| 91 | | 1.56 | |
| 95 | 25.00 | | |
| 96 | 12.50 | | |
| 100 | | 25.00 | |
| 101 | | 25.00 | |
| 102 | 1.56 | 0.39 | |
| 103 | 3.13 | 3.13 | |
| 104 | 3.13 | 0.39 | |
| 107 | 25.00 | 12.50 | |
| 108 | 0.39 | 3.13 | |
| 109 | 3.13 | | |
| 110 | 3.13 | | |
| 111 | 1.56 | 12.50 | |
| 112 | 12.50 | 12.50 | |
| 113 | 25.00 | 3.13 | |
| 114 | 6.25 | 3.13 | |

Example 117

The fungicidal activity of compounds of the invention was investigated by means of the following tests.

(a) Direct protectant activity against tomato late blight (Phytophthora infestans: PIP)

The test is a direct protectant one using a foliar spray. The upper leaf surfaces of tomato plants with two expanded leaves (cv. First in the field) are sprayed with a solution of active material in 1:1 water/acetone containing 0.04% "TWEEN 20" (Trade Mark; a polyoxyethylene sorbitan ester surfactant). Plants are treated using an automated sprayline with an atomising nozzle. The concentration of the compound is 1000 ppm, and the spray volume is 700 1/ha. After a subsequent period of 24 hours under normal glasshouse conditions, the upper surfaces of the leaves are inoculated by spraying with an aqueous suspension containing 2 x $10^5$ zoospores/ml. The inoculated plants are kept for 24 hours in a high humidity cabinet and 5 days under growth chamber conditions. The assessment is based on the percentage of diseased leaf area compared with that on control leaves.

(b) Direct protectant activity against vine downy mildew (Plasmopara viticola: PVP)

The test is a direct protectant one using a foliar spray. The lower surface of leaves of whole vine plants (cv Cabernet Sauvignon) are sprayed with the test compound at a dosage of 1000 ppm using an automated sprayline as described under (a), and after a subsequent period of 24 hours under normal glasshouse conditions the lower surfaces of the leaves are inoculated by spraying with an aqueous suspension containing 2.5 x $10^4$ zoosporangia/ml. The inoculated plants are kept for 24 hours in a high humidity cabinet, 5 days under normal glasshouse conditions and then returned for a further 24 hours to high humidity. Assessment is based on the percentage of leaf area covered by sporulation compared with that on control leaves.

(c) Activity against tomato early blight (Alternaria solani; AS)

This test measures the contact prophylactic activity of test compounds applied as a foliar spray. Tomato seedlings (cv Outdoor Girl) are grown to the stage at which the second true leaf is expanded. The plants are treated using an automated sprayline as described under (a). Test compounds are applied as solutions or suspensions in a mixture of acetone and water (50:50 v/v) containing 0.04% surfactant ("TWEEN 20" - Trade Mark). One day after treatment the seedlings are inoculated by spraying the leaf upper surfaces with a suspension of A. solani conidia containing $10^4$ spores/ml. For 4 days after inoculation plants are kept moist in a humidity compartment at 21°C. Disease is assessed 4 days after inoculation, based on the percentage of leaf surface area covered by lesions.

(d) Direct protectant activity against broad bean grey mould (Botrytis cinerea; BCB)

The test is a direct protectant one using a foliar spray. The upper surfaces of leaves of broad bean plants (cv The Sutton) are sprayed with the test compound at a dosage of 1000 ppm using an automated sprayline as described under (a). 24 hours after spraying the leaves are inoculated with an aqueous suspension containing $10^5$ conidia/ml. For 4 days after inoculation plants are kept moist in a humidity compartment at 21°C. Disease is assessed 4 days after inoculation, based on the percentage of leaf surface area covered by lesions.

(e) Activity against wheat leafspot (Leptosphaeria nodorum; LN.)

The test is a direct therapeutic one, using a foliar spray. Leaves of wheat plants (cv Norman), at the single leaf stage, are inoculated by spraying with an aqueous suspension containing 1 x $10^6$ spores/ml. The inoculated plants are kept for 24 hours in a high humidity compartment prior to treatment. The plants are sprayed with a solution of the test compound at a dosage of 1000 ppm using an automated sprayline as described under (a). After drying, the plants are kept for 6-8 days at 22°C and moderate humidity, followed by assessment. Assessment is based on the density of lesions per leaf compared with that on leaves of control plants.

(f) Activity against wheat brown rust (Puccinia recondita; PR)

The test is a direct protectant one using a foliar spray. Wheat seedlings (cv Avalon) are grown to the 1-1½ leaf stage. The plants are then sprayed with the test compound at a dosage of 1000 ppm using an automated sprayline as described under (a). Test compounds are applied as solutions or suspensions in a mixture of acetone and water (50:50 v/v) containing 0.04% surfactant ("TWEEN 20" - Trade Mark). 18-24 hours after treatment, the seedlings are inoculated by spraying the plants from all sides with an aqueous spore suspension containing about $10^5$ spores/ml. For 18 hours after inoculation, the plants are kept in high humidity conditions at a temperature of 20-22°C. Thereafter, the plants are kept in ambient glasshouse conditions, that is, in moderate relative humidity and at a temperature of 20°C. The disease is assessed 10 days after inoculation on the basis of the percentage of the plant covered by sporulating pustules compared with that on the control plants.

(g) Activity against barley powdery mildew (Erysiphe graminis f.sp. hordei; EG)

The test is a direct therapeutic one, using a foliar spray. Leaves of barley seedlings, (cv. Golden Promise) are inoculated by dusting with mildew conidia one day prior to treatment with the test compound. The inoculated plants are kept overnight at glasshouse ambient temperature and humidity prior to treatment. The plants are sprayed with the test compound at a dosage of 1000 ppm using an automated sprayline as described under (a). After drying, plants are returned to a compartment at 20-25°C and moderate humidity for up to 7 days, followed by assessment. Assessment is based on the percentage of leaf area covered by sporulation compared with that on leaves of control plants.

(h) Activity against rice leaf blast (Pyricularia oryzae; PO)

The test is a direct therapeutic one using a foliar spray. The leaves of rice seedlings (cv Aichiaishi - about 30 seedlings per pot) are sprayed with an aqueous suspension containing $10^5$ spores/ml 20-24 hours prior to treatment with the test compound. The inoculated plants are kept overnight in high humidity and then allowed to dry before spraying with the test compound at a dosage of 1000 ppm using an automated sprayline as described under (a). After treatment the plants are kept in a rice compartment at 25-30°C and high humidity. Assessments are made 4-5 days after treatment and are based on the density of necrotic lesions per leaf when compared with control plants.

(i) Activity against wheat eyespot in-vitro (Pseudocercosporella herpotrichoides; PHI)

This test measures the in vitro activity of compounds against the fungus causing wheat eyespot. The test compound is dissolved or suspended in acetone and is added into 4 ml aliquots of half strength Potato Dextrose Broth dispensed in 25-compartment petri dishes to give a final concentration of 50 ppm compound and 2.5% acetone. Each compartment is inoculated with a 6 mm diameter plug of agar/mycelium taken from a 14 day old

culture of P. herpotrichoides.

Plates are incubated at 20°C for 12 days until the assessment of mycelial growth.

(j) Activity against Fusarium in-vitro (Fusarium culmorum; FSI)

This test measures the in vitro activity of compounds against a species of Fusarium that causes stem and root rots. The test compound is dissolved or suspended in acetone and added to molten half strength Potato Dextrose Agar to give a final concentration of 50ppm compound and 2.5% acetone. After agar has set, plates are inoculated with 6mm diameter plugs of agar and mycelium taken from a 7 day old culture of Fusarium sp.. Plates are incubated at 20°C for 5 days and radial growth from the plug is measured.

(k) Activity against Rhizoctonia in-vitro (Rhizoctonia solani: RSI)

This test measures the in-vitro of compounds against Rhizoctonia solani that causes stem and root rots. The test compound is dissolved or suspended in acetone and added into aliquots of 4 ml half strength Potato Dextrose Broth dispensed in 25-compartment petri dishes to give a final concentration of 50 ppm compound and 2.5% acetone.

The fungal inoculum consists of mycelial fragments of R. solani grown in shaken culture flasks and added to the broth to provide 2 x $10^3$ fragments/ml broth.

Plates are incubated at 20°C for 10 days until the assessment of mycelial growth.

The extent of disease control in all the above tests is expressed as a rating compared with either an untreated control or a diluent-sprayed-control, according to the criteria:-

0 = less than 50% disease control
1 = about 50-80% disease control
2 = greater than 80% disease control

The results of these tests are set out in Table IV below:-

TABLE IV

| Ex. | Fungicidal Activity | | | | | | | | | | |
|---|---|---|---|---|---|---|---|---|---|---|---|
| No. | PIP | PVP | AS | BCB | LN | PR | EG | PO | PHI | FSI | RSI |
| 9 | | 1 | 2 | 1 | | | 1 | | | | |
| 33 | | | | 2 | | | | | | | |
| 36 | | | 2 | | | 2 | 1 | | | 1 | |
| 37 | 1 | | | 2 | | 2 | 2 | | | 1 | |
| 38 | 1 | | 2 | 2 | | | | | | | |
| 39 | | | 2 | 2 | | | 2 | | 1 | | 2 |
| 40 | | | 2 | 2 | 2 | | 2 | 2 | 1 | | 2 |
| 41 | | | | 2 | | 1 | | | | 1 | |
| 42 | | | 2 | 2 | | 1 | 2 | 2 | 1 | | |
| 43 | | | | 2 | | 1 | | | 1 | | |
| 52 | | 1 | 2 | 1 | | | | | | | |
| 53 | | | 1 | | | | | | | | |

TABLE IV (continued)

| Ex. No. | Fungicidal Activity PIP | PVP | AS | BCB | LN | PR | EG | PO | PHI | FSI | RSI |
|---|---|---|---|---|---|---|---|---|---|---|---|
| 64 | | | 2 | 1 | | 1 | | | | | |
| 65 | | | | 2 | | 1 | 1 | | 1 | 1 | |
| 66 | | | 2 | | | | | | 2 | | |
| 67 | | | 2 | | | 2 | | | | | |
| 68 | | | | | | | 1 | | | | |
| 69 | | | 2 | 2 | | 1 | | | 2 | 1 | |
| 70 | | | 2 | 1 | | 2 | 1 | | 2 | | |
| 71 | | | | | | 2 | | | | | |
| 72 | | | 2 | | | | | | | | |
| 73 | | | | | | 1 | | | | | |
| 74 | | | | | | 1 | | | | | |
| 75 | | | | | | 1 | | | | | |
| 76 | | | 2 | 1 | | 1 | | | | | |
| 77 | | | 2 | 2 | | 1 | | | | | |
| 78 | | | 1 | 1 | | | | | | | |
| 81 | | | 2 | 2 | | | | | 2 | | 2 |
| 83 | | | | | | | | | | | 1 |
| 84 | | | 2 | 1 | | | | | 1 | | |
| 85 | | | 1 | | | | | | 1 | | |

Example 118

The fungicidal activity of compounds of the invention was investigated by means of the following tests.

(a) Antisporulant activity against vine downy mildew (Plasmopara viticola; PVA)

The test is a direct antisporulant one using a foliar spray. The lower surface of leaves of vine plants (cv. Cabernet Sauvignon), approximately 8cm high, are inoculated with an aqueous suspension containing 5 x $10^4$ zoosporangia/ml. The inoculated plants are kept for 24 hours at 21°C in a high humidity cabinet, then for 24 hours in a glasshouse at 20°C and 40% relative humidity. Infected leaves are sprayed on their lower surfaces with a solution of the test compound in 1:1 water/acetone containing 0.04% "TWEEN 20" (Trade Mark; a polyoxyethylene sorbitan ester surfactant). Plants are sprayed using a track sprayer equipped with 2 air-atomising nozzles. The concentration of the compound is 600 ppm and the spray volume is 750 l/ha. After drying, the plants are returned to the glasshouse at 20°C and 40% relative humidity for 96 hours and are then transferred to the high humidity cabinet for 24 hours to induce sporulation. Assessment is based on the percentage of the leaf area covered by sporulation compared with that on control leaves.

(b) Direct protectant activity against tomato late blight (Phytophthora infestans; PIP)

The test is a direct protectant one using a foliar spray. Tomato plants with two expanded leaves (cv. First in the Field) are sprayed with the test compound at a dosage of 600 ppm as described under (a). After drying, the plants are kept for 24 hours in a glasshouse at 20°C and 40% relative humidity. The upper surfaces of the leaves

are then inoculated with an aqueous suspension containing 2 x $10^5$ zoosporangia/ml. The inoculated plants are kept for 24 hours at 18°C in a high humidity cabinet and then for 5 days in a growth chamber at 15°C and 80% relative humidity with 14 hours light/day. The assessment is based on the percentage of diseased leaf area compared with that on control leaves.

(c) Activity against tomato early blight (Alternaria solani; AS)

The test is a direct prophylactic one using a foliar spray. Tomato seedlings (cv Outdoor Girl), at the stage at which the second leaf is expanded, are sprayed with the test compound at a dosage of 600ppm as described under (a). After drying, the plants are kept for 24 hours in a glasshouse at 20°C and 40% relative humidity followed by inoculation of the leaf upper surfaces with an aqueous suspension of A. solani conidia containing 1 x $10^4$ conidia/ml. After 4 days in a high humidity cabinet at 21°C, disease is assessed based on the percentage of leaf surface area covered by lesions when compared with control plants.

(d) Direct protectant activity against broad bean grey mould (Botrytis cinerea; BCB)

The test is a direct protectant one using a foliar spray. Broad bean plants (cv The Sutton) with two leaf pairs are sprayed with the test compound at a dosage of 600 ppm as described under (a). After drying, the plants are kept for 24 hours in a glasshouse at 20°C and 40% relative humidity. The upper surface of the leaves are then inoculated with an aqueous suspension containing 1 x $10^6$ conidia/ml. Plants are kept for 4 days at 22°C in a high humidity cabinet. The assessment is based on the percentage of diseased leaf area compared with that on control leaves.

(e) Activity against barley powdery mildew (Erysiphe graminis f.sp. hordei: EG)

The test is a direct therapeutic one using a foliar spray. Leaves of barley seedlings (cv Golden Promise) at the single leaf stage are inoculated by dusting with mildew conidia and kept in the glasshouse at 18°C and 40% relative humidity for 24 hours. Plants are then sprayed with the test compound at a dosage of 600 ppm as described under (a). After drying, plants are returned to the glasshouse at 18°C and 40% relative humidity for up to 7 days. Assessment is based on the percentage of leaf area covered by sporulation compared with that on leaves of control plants.

(f) Activity against rice leaf blast (Pyricularia oryzae; PO)

The test is a direct therapeutic one using a foliar spray. The leaves of rice seedlings at the stage of the second leaf beginning to bend (cv Aichiaishi) are inoculated with an aqueous suspension containing $10^5$ spores/ml. The inoculated plants are kept for 24 hours at 18°C in a high humidity cabinet and then sprayed with the test compound at a dosage of 600 ppm as described under (a). Treated plants are kept for 8-9 days in the glasshouse at 22°C and 90% relative humidity. Assessment is based on the density of necrotic lesions when compared with control plants.

(g) Activity against wheat eyespot in-vitro (Pseudocercosporella herpotrichoides; PHI)

This test measures the in vitro activity of compounds against the fungus causing wheat eyespot. The test compound is dissolved or suspended in acetone and is added into 4 ml aliquots of half strength Potato Dextrose Broth dispensed in 25-compartment petri dishes to give a final concentration of 10 ppm test compound and 0.825% acetone. The fungal inoculum consists of mycelial fragments of P. herpotrichoides grown in half strength Potato Dextrose Broth in shaken flasks and added to the broth to provide 5 x $10^4$ mycelial fragments/ml broth. Petri dishes are incubated at 20°C for 10 days until the assessment of mycelial growth.

(h) Activity against Rhizoctonia in-vitro (Rhizoctonia solani: RSI)

The test measures the in-vitro activity of compounds against Rhizoctonia solani that causes stem and root rots. The test compound is dissolved or suspended in acetone and added into 4ml aliquots of half strength Potato Dextrose Broth dispensed in 25-compartment petri dishes to give a final concentration of 10 ppm compound and 0.825% acetone. The fungal inoculum consists of mycelial fragments of R. solani grown in half strength Potato Dextrose Broth in shaken culture flasks and added to the broth to provide 5 x $10^4$ fragments/ml broth. Petri dishes are incubated at 20°C for 10 days until the assessment of mycelial growth.

(i) Activity against apple scab in-vitro (Venturia inaequalis; VII)

This test measures the in-vitro activity of compounds against Venturia inaequalis that causes apple scab. The test compound is dissolved or suspended in acetone and added into 4ml aliquots of half strength Potato Dextrose Broth dispensed in 25-compartment petri dishes to give a final concentration of 10ppm compound and 0.825% acetone. The fungal inoculum consists of mycelial fragments and spores of V. inaequalis grown on malt agar and added to the broth to provide 5 x $10^4$ propagules/ml broth. Petri dishes are incubated at 20°C for 10 days until the assessment of mycelial growth.

The extent of disease control in all the above tests is expressed as a rating compared with either an untreated control or a diluent-sprayed-control, according to the criteria:-

0 = less than 50% disease control

1 = 50-80% disease control
2 = greater than 80% disease control

The results of these tests are set out in Table V below:-

TABLE V

| Example | Fungicidal Activity | | | | | | | | |
|---|---|---|---|---|---|---|---|---|---|
| No. | PVA | PIP | AS | BCB | EG | PO | PHI | RSI | VII |
| 44 | | | | | | | | | 2** |
| 86 | | | 1 | 1 | | | | | 1* |
| 87 | | 1 | 2 | | | | | | 1* |
| 89 | 1 | | | | | 1 | | | |
| 90 | | 2 | | | | | | | |
| 91 | | | 2 | | | | | 1 | 2 |
| 92 | | | 2 | | | | | 1 | 2 |
| 93 | | | 2 | | | | | | 2 |
| 94 | | 2 | | | | | | | |
| 95 | | 2 | 2 | 1 | | | | 1 | 1 |
| 96 | | 2 | 2 | 2 | 1 | | | 2 | 2 |

TABLE V (continued)

| Example No. | Fungicidal Activity | | | | | | | | |
|---|---|---|---|---|---|---|---|---|---|
| | PVA | PIP | AS | BCB | EG | PO | PHI | RSI | VII |
| 97 | | 2 | | | | | | | |
| 98 | | | 2 | | | | | 1 | |
| 99 | | 2 | 2 | | 1 | | | 1 | 2 |
| 103 | | 2 | 2 | 2 | 2 | 1 | 1 | 2 | 1 |
| 104 | | 2 | 2 | 2 | 2 | | 1 | 2 | 1 |
| 105 | | 2 | | | | | | | |
| 106 | | 2 | 2 | 2 | 1 | | | 2 | |

* signifies dosage of test compound - 30 ppm

** " " " " " - 3 ppm

## Claims

1. A compound of the general formula

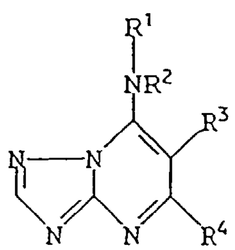

(I)

in which

$R^1$ represents an optionally substituted $C_{1-12}$ alkyl, $C_{2-12}$ alkenyl, $C_{2-12}$ alkynyl, $C_{4-12}$ alkadienyl, $C_{3-8}$ cycloalkyl, $C_{4-12}$ bicycloalkyl or 3- to 6-membered heterocyclyl group;
$R^2$ represents a hydrogen atom or a $C_{1-12}$ alkyl group; or
$R^1$ and $R^2$ together with the interjacent nitrogen atom represent an optionally substituted 3- to 6-membered heterocyclic ring;
$R^3$ represents an optionally substituted phenyl or naphthyl group; and
$R^4$ represents a halogen atom or a group $-NR^5R^6$ where $R^5$ represents a hydrogen atom or an amino, $C_{1-12}$ alkyl, $C_{3-8}$ cycloalkyl or $C_{4-12}$ bicycloalkyl group and $R^6$ represents a hydrogen atom or a $C_{1-12}$ alkyl group;
optional substituents being selected from halogen atoms, nitro, cyano, thiocyanato, cyanato, hydroxyl, $C_{1-12}$ alkyl, $C_{1-12}$ haloalkyl, $C_{1-12}$ alkoxy, $C_{1-12}$ haloalkoxy, amino, $C_{1-12}$ alkylamino, di-$C_{1-12}$ alkylamino, formyl, $C_{1-12}$ alkoxycarbonyl, carboxyl, $C_{1-12}$ alkanoyl, $C_{1-12}$ alkylthio, $C_{1-12}$ alkylsulphinyl, $C_{1-12}$ alkylsulphonyl, carbamoyl,

$C_{1-12}$ alkylamido and 3- to 6-membered heterocyclyl groups and optionally substituted phenyl, phenoxy, benzyl, benzyloxy and $C_{3-8}$ cycloalkyl groups each optionally substituted by one or more halogen atoms, nitro, cyano, $C_{1-12}$ alkyl, $C_{1-12}$ haloalkyl, $C_{1-12}$ alkoxy or $C_{1-12}$ haloalkoxy groups, and additional optional substituents in the case of cycloalkyl and heterocyclyl groups being selected from saturated and unsaturated hydrocarbyl rings which may be fused with the cycloalkyl or heterocyclyl group.

2. A compound according to claim 1 in which $R^1$ represents a $C_{1-12}$ alkyl, $C_{2-6}$ alkenyl, $C_{2-6}$ alkynyl, $C_{4-12}$ alkadienyl, $C_{3-8}$ cycloalkyl or $C_{4-8}$ bicycloalkyl group or a 3- to 6-membered heterocyclic ring, each group or ring being optionally substituted by one or more substituents selected from halogen atoms, nitro, cyano, hydroxyl, $C_{1-4}$ alkyl, $C_{1-4}$ haloalkyl, $C_{1-4}$ alkoxyl, $C_{1-4}$ haloalkoxy, amino. $C_{1-4}$ alkylamino, di-$C_{1-4}$ alkylamino, formyl, $C_{1-4}$ alkoxycarbonyl, carboxyl, phenyl, $C_{1-4}$ haloalkylphenyl, di-$C_{1-4}$ alkoxyphenyl, furyl and dihalo-$C_{3-6}$ cycloalkyl groups or, in the case where $R^1$ represents a $C_{3-8}$ cycloalkyl group or a 3- to 6-membered heterocyclic ring, optionally ortho-fused with a benzene ring.

3. A compound according to claim 1 or claim 2 in which $R^2$ represents a hydrogen atom or a $C_{1-4}$ alkyl group.

4. A compound according to any one of the preceding claims in which $R^3$ represents a phenyl or naphthyl group, each group being optionally substituted by one or more substituents selected from halogen atoms, nitro, cyano, hydroxyl, $C_{1-12}$ alkyl, $C_{1-12}$ haloalkyl, $C_{1-12}$ alkoxy, $C_{1-12}$ haloalkoxy, amino, $C_{1-4}$ alkylamino, di-$C_{1-4}$ alkylamino, formyl, $C_{1-4}$ alkoxycarbonyl, carboxyl, phenyl, phenoxy and benzyloxy groups.

5. A compound according to any one of the preceding claims in which $R^4$ represents a halogen atom or a group $-NR^5R^6$ where $R^5$ represents a hydrogen atom or an amino, $C_{1-4}$ alkyl, $C_{3-6}$ cycloalkyl or $C_{4-8}$ bicycloalkyl group and $R^6$ represents a hydrogen atom or a $C_{1-4}$ alkyl group.

6. A compound according to any one of the preceding claims in which $R^1$ represents a methyl, ethyl, propyl, heptyl, dodecyl, benzyl, dichlorocyclopropylmethyl, furylmethyl, trifluoromethylphenethyl, dimethoxyphenethyl, pentenyl, propynyl, dimethyloctadienyl, cyclopropyl, cyclopentyl, hydroxycyclopentyl, trimethylcyclopentyl, cyclohexyl, trimethylcyclohexyl, cyclooctyl, indanyl, bicycloheptyl, dichloroaziridinyl, pyrrolidinyl, morpholinyl or benzothiazolyl group; $R^2$ represents a hydrogen atom, methyl or ethyl group; or $R^1$ and $R^2$ together with the interjacent nitrogen atom represent a phenylpiperidyl group; $R^3$ represents a phenyl, fluorophenyl, chlorophenyl, bromophenyl, chloro-fluorophenyl, methylphenyl, propylphenyl, trifluoromethylphenyl, methoxyphenyl, ethoxyphenyl, dimethoxyphenyl, trimethoxyphenyl, trifluoromethoxyphenyl, biphenylyl, phenoxyphenyl, benzyloxyphenyl or naphthyl group; and $R^4$ represents a fluorine, chlorine, bromine or iodine atom or an amino, methylamino, dimethylamino, hydrazino, cyclopentylamino or bicycloheptylamino group.

7. A process for the preparation of a compound of formula I as defined in any one of the preceding claims which comprises

(a) reacting a compound of the general formula

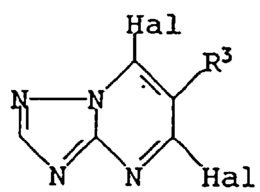

(II)

in which $R^3$ is as defined in any one of the preceding claims and Hal represents a chlorine or bromine atom, with a compound of the general formula

$$HNR^1R^2 \quad (III)$$

in which $R^1$ and $R^2$ are as defined in any one of the preceding claims, to produce a compound of formula I in

which $R^4$ represents a chlorine or bromine atom;
(b) if desired, reacting the compound of formula I formed in (a) with a fluorinating agent to produce a compound of formula I in which $R^4$ represents a fluorine atom;
(c) if desired, reacting the compound of formula I formed in (a) with a compound of the general formula

$$HNR^5R^6 \quad (IV)$$

in which $R^5$ and $R^6$ are as defined in any one of the preceding claims, to produce a compound of formula I in which $R^4$ represents a group $-NR^5R^6$; and
(d) if desired, reacting a compound of formula I formed in (c) in which $R^5$ and $R^6$ both represent a hydrogen atom with diiodomethane in the presence of a diazotising agent to produce a compound of formula I in which $R^4$ represents an iodine atom.

**8.** A fungicidal composition which comprises a carrier and, as active ingredient, a compound of formula I as defined in any one of claims 1 to 6.

**9.** A method of combating fungus at a locus which comprises treating the locus with a compound of formula I as defined in any one of claims 1 to 6 or with a composition as defined in claim 8.

**10.** The use as a fungicide of a compound of formula I as defined in any one of claims 1 to 6 or a composition as defined in claim 8.

## Patentansprüche

**1.** Verbindung der allgemeinen Formel

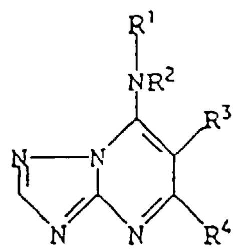

(I)

worin

$R^1$ eine gegebenenfalls substituierte $C_{1-12}$-Alkyl-, $C_{2-12}$-Alkenyl-, $C_{4-12}$-Alkadienyl-, $C_{3,8}$-Cycloalkyl-, $C_{4-12}$-Bicycloalkyl- oder 3- bis 6-gliedrige heterocyclische Gruppe darstellt;
$R^2$ ein Wasserstoffatom oder eine $C_{1-12}$-Alkylgruppe darstellt; oder
$R^1$ und $R^2$ zusammen mit dem dazwischenliegenden Stickstoffatom einen gegebenenfalls substituierten 3- bis 6-gliedrigen heterocyclischen Ring wiedergeben;
$R^3$ eine gegebenenfalls substituierte Phenyl- oder Naphthylgruppe wiedergibt; und
$R^4$ ein Halogenatom oder eine Gruppe $-NR^5R^6$ wiedergibt, worin $R^5$ ein Wasserstoffatom oder eine Amino-, $C_{1-12}$-Alkyl-, $C_{3-8}$-Cycloalkyl- oder $C_{4-12}$-Bicycloalkylgruppe wiedergibt und $R^6$ ein Wasserstoffatom oder eine $C_{1-12}$-Alkylgruppe wiedergibt;

wobei gegebenenfalls vorliegende Substituenten ausgewählt sind aus Halogenatomen, Nitro-, Cyano-, Thiocyanat-, Cyanat-, Hydroxyl-, $C_{1-12}$-Alkyl-, $C_{1-12}$-Halogenalkyl-, $C_{1-12}$-Alkoxy-, $C_{1-12}$-Halogenalkoxy-, Amino-, $C_{1-12}$-Alkylamino-, Di-$C_{1-12}$-alkylamino-, Formyl-, $C_{1-12}$-Alkoxycarbonyl-, Carboxyl-, $C_{1-12}$-Alkanoyl-, $C_{1-12}$-Alkylthio-, $C_{1-12}$-Alkylsulfinyl-, $C_{1-12}$-Alkylsulfonyl-, Carbamoyl-, $C_{1-12}$-Alkylamido- und 3- bis 6-gliedrigen heterocyclischen Gruppen und gegebenenfalls substituierten Phenyl-, Phenoxy-, Benzyl-, Benzyloxy- und $C_{3-8}$-Cycloalkylgruppen, jeweils gegebenenfalls substituiert mit einem oder mehreren Halogenatomen, Nitro-, Cyano-, $C_{1-12}$-Alkyl-, $C_{1-12}$-Halogenalkyl-, $C_{1-12}$-Alkoxy- oder $C_{1-12}$-Halogenalkoxygruppen und weiteren, gegebenenfalls vorliegenden Substituenten im Fall von Cycloalkyl- und Heterocyclylgruppen, ausgewählt aus gesättigten und ungesättigten Kohlenwasserstoffringen, die mit der Cycloalkyl- oder Heterocyclylgruppe kondensiert sein können.

2. Verbindung nach Anspruch 1, worin $R^1$ eine $C_{1-12}$-Alkyl-, $C_{2-6}$-Alkenyl-, $C_{2-6}$-Alkinyl-, $C_{4-12}$-Alkadienyl-, $C_{3-8}$-Cycloalkyl- oder $C_{4-8}$-Bicycloalkylgruppe oder einen 3- bis 6-gliedrigen heterocyclischen Ring wiedergibt, wobei jede Gruppe oder jeder Ring gegebenenfalls mit einem oder mehreren Substituenten, ausgewählt aus Halogenatomen, Nitro-, Cyano-, Hydroxyl-, $C_{1-4}$-Alkyl-, $C_{1-4}$-Halogenalkyl-, $C_{1-4}$-Alkoxy-, $C_{1-4}$-Halogenalkoxy-, Amino-, $C_{1-4}$-Alkylamino-, Di-$C_{1-4}$-alkylamino-, Formyl-, $C_{1-4}$-Alkoxycarbonyl-, Carboxyl-, Phenyl-, $C_{1-4}$-Halogenalkylphenyl-, Di-$C_{1-4}$-alkoxyphenyl-, Furyl- und Dihalogen-$C_{3-6}$-cycloalkylgruppen substituiert ist oder im Fall, wenn $R^1$ eine $C_{3-8}$-Cycloalkylgruppe oder einen 3- bis 6-gliedrigen heterocyclischen Ring wiedergibt, gegebenenfalls mit einem Benzolring in ortho-Stellung kondensiert ist.

3. Verbindung nach Anspruch 1 oder Anspruch 2, worin $R^2$ ein Wasserstoffatom oder eine $C_{1-4}$-Alkylgruppe wiedergibt.

4. Verbindung nach einem der vorangehenden Ansprüche, worin $R^3$ eine Phenyl- oder Naphthylgruppe wiedergibt, wobei jede Gruppe gegebenenfalls mit einem oder mehreren Substituenten, ausgewählt aus Halogenatomen, Nitro-, Cyano-, Hydroxyl-, $C_{1-12}$-Alkyl-, $C_{1-12}$-Halogenalkyl-, $C_{1-12}$-Alkoxy-, $C_{1-12}$-Halogenalkoxy-, Amino-, $C_{1-4}$-Alkylamino-, Di-$C_{1-4}$-alkylamino-, Formyl-, $C_{1-4}$-Alkoxycarbonyl-, Carboxyl-, Phenyl-, Phenoxy- und Benzyloxygruppen substituiert ist.

5. Verbindung nach einem der vorangehenden Ansprüche, worin $R^4$ ein Halogenatom oder eine Gruppe $-NR^5R^6$ wiedergibt, worin $R^5$ ein Wasserstoffatom oder eine Amino-, $C_{1-4}$-Alkyl-, $C_{3-6}$-Cycloalkyl- oder $C_{4-8}$-Bicycloalkylgruppe wiedergibt und $R^6$ ein Wasserstoffatom oder eine $C_{1-4}$-Alkylgruppe wiedergibt.

6. Verbindung nach einem der vorangehenden Ansprüche, worin $R^1$ eine Methyl-, Ethyl-, Propyl-, Heptyl-, Dodecyl-, Benzyl-, Dichlorcyclopropylmethyl-, Furylmethyl-, Trifluormethylphenethyl-, Dimethoxyphenethyl-, Pentenyl-, Propinyl-, Dimethyloctadienyl-, Cyclopropyl-, Cyclopentyl-, Hydroxycyclopentyl-, Trimethylcyclopentyl-, Cyclohexyl-, Trimethylcyclohexyl-, Cyclooctyl-, Indanyl-, Bicycloheptyl-, Dichloraziridinyl-, Pyrrolidinyl-, Morpholinyl- oder Benzothiazolylgruppe wiedergibt; $R^2$ ein Wasserstoffatom, eine Methyl- oder Ethylgruppe wiedergibt; oder $R^1$ und $R^2$ zusammen mit dem dazwischenliegenden Stickstoffatom eine Phenylpiperidylgruppe wiedergeben; $R^3$ eine Phenyl-, Fluorphenyl-, Chlorphenyl-, Bromphenyl-, Chlorfluorphenyl-, Methylphenyl-, Propylphenyl-, Trifluormethylphenyl-, Methoxyphenyl-, Ethoxyphenyl-, Dimethoxyphenyl-, Trimethoxyphenyl-, Trifluormethoxyphenyl-, Biphenylyl-, Phenoxyphenyl-, Benzyloxyphenyl- oder Naphthylgruppe wiedergibt; und $R^4$ ein Fluor-, Chlor-, Brom- oder Jodatom oder eine Amino-, Methylamino-, Dimethylamino-, Hydrazino-, Cyclopentylamino- oder Bicycloheptylaminogruppe wiedergibt.

7. Verfahren zur Herstellung einer, wie in einem der vorangehenden Ansprüche definierten Verbindung der Formel I, umfassend

(a) Umsetzen einer Verbindung der allgemeinen Formel

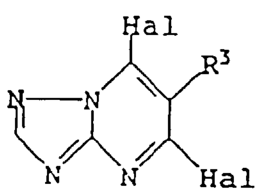

(II)

worin $R^3$ wie in einem der vorangehenden Ansprüche definiert ist und Hal ein Chlor- oder Bromatom wiedergibt, mit einer Verbindung der allgemeinen Formel

$$HNR^1R^2 \quad (III),$$

worin $R^1$ und $R^2$ wie in einem der vorangehenden Ansprüche definiert sind, zur Herstellung einer Verbindung der Formel I, worin $R^4$ ein Chlor- oder Bromatom wiedergibt;
(b) falls erwünscht, Umsetzen der in (a) gebildeten Verbindung der Formel I mit einem Fluorierungsmittel, zur Herstellung einer Verbindung der Formel I, worin $R^4$ ein Fluoratom wiedergibt;
(c) falls erwünscht, Umsetzen der in (a) gebildeten Verbindung der Formel I mit einer Verbindung der allgemeinen Formel

$$HNR^5R^6 \qquad (IV),$$

worin $R^5$ und $R^6$ wie in einem der vorangehenden Ansprüche definiert sind, zur Herstellung einer Verbindung der Formel I, worin $R^4$ eine Gruppe $-NR^5R^6$ wiedergibt; und
(d) falls erwünscht, Umsetzen einer in (c) gebildeten Verbindung der Formel I, worin $R^5$ und $R^6$ beide ein Wasserstoffatom wiedergeben, mit Dijodmethan, in Gegenwart eines diazotierenden Mittels, zur Herstellung einer Verbindung der Formel I, worin $R^4$ ein Jodatom wiedergibt.

**8.** Fungizides Mittel, umfassend einen Träger und als Wirkstoff eine wie in einem der Ansprüche 1 bis 6 definierte Verbindung der Formel I.

**9.** Verfahren zur Bekämpfung von Pilz an einem Ort, umfassend Behandeln des Orts mit einer wie in einem der Ansprüche 1 bis 6 definierten Verbindung der Formel I oder mit einem wie in Anspruch 8 definierten Mittel.

**10.** Verwendung einer wie in einem der Ansprüche 1 bis 6 definierten Verbindung der Formel I oder eines wie in Anspruch 8 definierten Mittels als Fungizid.

## Revendications

**1.** Composé de formule générale :

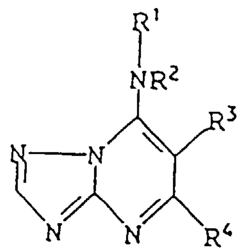

(I)

dans laquelle

$R^1$ représente un groupe alkyle en $C_1$-$C_{12}$, alcényle en $C_2$-$C_{12}$, alcynyle en $C_2$-$C_{12}$, alcadiényle en $C_4$-$C_{12}$, cycloalkyle en $C_3$-$C_8$, bicycloalkyle en $C_4$-$C_{12}$ ou hétérocyclique à 3 jusqu'à 6 chaînons, éventuellement substitué ;
$R^2$ représente un atome d'hydrogène ou un groupe alkyle en $C_1$-$C_{12}$ ; ou
$R^1$ et $R^2$ représentent ensemble avec l'atome d'azote adjacent, un hétérocycle à 3 jusqu'à 6 chaînons ;
$R^3$ représente un groupe phényle ou naphtyle, éventuellement substitué ; et
$R^4$ représente un atome d'halogène ou un groupe $-NR^5R^6$ dans lequel $R^5$ représente un atome d'hydrogène ou un groupe amino, un groupe alkyle en $C_1$-$C_{12}$, un groupe cycloalkyle en $C_3$-$C_8$ ou bicycloalkyle en $C_4$-$C_{12}$, et $R^6$ représente un atome d'hydrogène ou un groupe alkyle en $C_1$-$C_{12}$ ;
les substituants facultatifs étant choisis parmi les atomes d'halogène, les groupes nitro, cyano, thiocyanato, cyanato, hydroxyle, alkyle en $C_1$-$C_{12}$, haloalkyle en $C_1$-$C_{12}$, alkoxy en $C_1$-$C_{12}$, haloalkoxy en $C_1$-$C_{12}$, amino, alkylamino en $C_1$-$C_{12}$, di-(alkyl en $C_1$-$C_{12}$)amino, formyle, (alkoxy en $C_1$-$C_{12}$)carbonyle, carboxyle, alcanoyle en $C_1$-$C_{12}$, alkylthio en $C_1$-$C_{12}$, alkylsulfinyle en $C_1$-$C_{12}$, alkylsulfonyle en $C_1$-$C_{12}$, carbamoyle, alkylamido en $C_1$-$C_{12}$ et hétérocycliques à 3 jusqu'à 6 chaînons, et les groupes phényle, phénoxy, benzyle, benzyloxy et

cycloalkyle en $C_3$-$C_8$ éventuellement substitués, chacun étant éventuellement substitué avec un ou plusieurs atomes d'halogène, groupes nitro, cyano, alkyle en $C_1$-$C_{12}$, haloalkyle en $C_1$-$C_{12}$, alkoxy en $C_1$-$C_{12}$ ou haloalkoxy en $C_1$-$C_{12}$, et d'autres substituants facultatifs supplémentaires dans le cas des groupes cycloalkyle et hétérocycliques étant choisis parmi les noyaux hydrocarbonés saturés et insaturés qui peuvent être condensés avec le groupe cycloalkyle ou hétérocyclique.

**2.** Composé selon la revendication 1, dans lequel $R^1$ représente un groupe alkyle en $C_1$-$C_{12}$, alcényle en $C_2$-$C_6$, alcynyle en $C_2$-$C_6$, alcadiényle en $C_4$-$C_{12}$, cycloalkyle en $C_3$-$C_8$ ou bicycloalkyle en $C_4$-$C_8$, ou un hétérocycle à 3 jusqu'à 6 chaînons, chaque groupe ou cycle étant éventuellement substitué avec un ou plusieurs substituants choisis parmi les atomes d'halogène, les groupes nitro, cyano, hydroxy, alkyle en $C_1$-$C_4$, haloalkyle en $C_1$-$C_4$, alkoxy en $C_1$-$C_4$, haloalkoxy en $C_1$-$C_4$, amino, alkylamino en $C_1$-$C_4$, di-(alkyl en $C_1$-$C_4$)amino, formyle, (alkoxy en $C_1$-$C_4$)carbonyle, carboxyle, phényle, (haloalkyl en $C_1$-$C_4$)phényle, di-(alkoxy en $C_1$-$C_4$)phényle, furyle et dihalo-(cycloalkyl en $C_3$-$C_6$), ou dans le cas où $R^1$ représente un groupe cycloalkyle en $C_3$-$C_8$ ou un hétérocycle à 3 jusqu'à 6 chaînons, éventuellement ortho-condensé avec un cycle benzénique.

**3.** Composé selon la revendication 1 ou 2, dans lequel $R^2$ représente un atome d'hydrogène ou un groupe alkyle en $C_1$-$C_4$.

**4.** Composé selon l'une quelconque des revendications précédentes, dans lequel $R^3$ représente un groupe phényle ou naphtyle, chaque groupe étant éventuellement substitué avec un ou plusieurs substituants choisis parmi les atomes d'halogène, les groupes nitro, cyano, hydroxyle, alkyle en $C_1$-$C_{12}$, haloalkyle en $C_1$-$C_{12}$, alkoxy en $C_1$-$C_{12}$, haloalkoxy en $C_1$-$C_{12}$, amino, alkylamino en $C_1$-$C_4$, di-(alkyl en $C_1$-$C_4$)amino, formyle, ou (alkoxy en $C_1$-$C_4$)carbonyle, carboxyle, phényle, phénoxy et benzyloxy.

**5.** Composé selon l'une quelconque des revendications précédentes, dans lequel $R^4$ représente un atome d'halogène ou un groupe $-NR^5R^6$ dans lequel $R^5$ représente un atome d'hydrogène ou un groupe amino, un groupe alkyle en $C_1$-$C_4$, cycloalkyle en $C_3$-$C_6$ ou bicycloalkyle en $C_4$-$C_8$, et $R^6$ représente un atome d'hydrogène ou un groupe alkyle en $C_1$-$C_4$.

**6.** Composé selon l'une quelconque des revendications précédentes, dans lequel $R^1$ représente un groupe méthyle, éthyle, propyle, heptyle, dodécyle, benzyle, dichlorocyclopropylméthyle, furylméthyle, trifluorométhylphénéthyle, diméthoxyphénéthyle, pentényle, propynyle, diméthyloctadiényle, cyclopropyle, cyclopentyle, hydroxycyclopentyle, triméthylcyclopentyle, cyclohexyle, triméthylcyclohexyle, cyclooctyle, indanyle, bicycloheptyle, dichloroaziridinyle, pyrrolidinyle, morpholinyle ou benzothiazolyle ; $R^2$ représente un atome d'hydrogène, un groupe méthyle ou éthyle ; ou $R^1$ et $R^2$ forment ensemble avec l'atome d'azote adjacent, un groupe phénylpipéridyle ; $R^3$ représente un groupe phényle, fluorophényle, chlorophényle, bromophényle, chlorofluorophényle, méthylphényle, propylphényle, trifluorométhylphényle, méthoxyphényle, éthoxyphényle, diméthoxyphényle, triméthoxyphényle, trifluorométhoxyphényle, biphénylyle, phénoxyphényle, benzyloxyphényle ou naphtyle ; et $R^4$ représente un atome de fluor, de chlore, de brome ou d'iode, ou un groupe amino, méthylamino, diméthylamino, hydrazino, cyclopentylamino ou bicycloheptylamino.

**7.** Procédé de préparation d'un composé de formule I telle que définie dans l'une quelconque des revendications précédentes, selon lequel :

(a) on fait réagir un composé de formule générale :

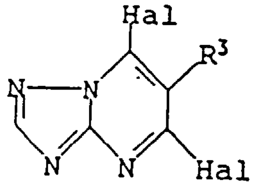

(II)

dans laquelle $R^3$ est tel que défini dans l'une quelconque des revendications précédentes, et Hal représente un atome de chlore ou de brome, avec un composé de formule générale :

$$HNR^1R^2 \qquad (III)$$

dans laquelle $R^1$ et $R^2$ sont tels que définis dans l'une quelconque des revendications précédentes, pour produire un composé de formule I dans laquelle $R^4$ représente un atome de chlore ou de brome ;
(b) on fait réagir, si on le souhaite, le composé de formule I formé en (a), avec un agent de fluoration, pour produire un composé de formule I dans laquelle $R^4$ représente un atome de fluor ;
(c) on fait réagir, si on le souhaite, le composé de formule I formé en (a), avec un composé de formule générale :

$$HNR^5R^6 \qquad (IV)$$

dans laquelle $R^5$ et $R^6$ sont tels que définis dans l'une quelconque des revendications précédentes, pour produire un composé de formule I dans laquelle $R^4$ représente un groupe $-NR^5R^6$ ; et
(d) on fait réagir, si on le souhaite, un composé de formule I formé en (c), dans lequel $R^5$ et $R^6$ représentent tous les deux un atome d'hydrogène, avec du diiodométhane en présence d'un agent de diazotation pour produire un composé de formule I dans laquelle $R^4$ représente un atome d'iode.

**8.** Composition fongicide comprenant un véhicule et, comme ingrédient actif, un composé de formule I tel que défini dans l'une quelconque des revendications 1 à 6.

**9.** Procédé pour lutter contre un champignon à un emplacement, selon lequel on traite l'emplacement avec un composé de formule I tel que défini dans l'une quelconque des revendications 1 à 6, ou avec une composition telle que définie dans la revendication 8.

**10.** Utilisation comme fongicide, d'un composé de formule I tel que défini dans l'une quelconque des revendications 1 à 6, ou d'une composition telle que définie dans la revendication 8.